# EUROPÄISCHE PATENTSCHRIFT

(11) **EP 4 638 411 B1**
(45) Veröffentlichungstag und Bekanntmachung des Hinweises auf die Patenterteilung: **23.09.2026**
(21) Anmeldenummer: 23834186.1
(22) Anmeldetag: 20.12.2023
(51) Int. Cl.: C07C 69/716, C08K 5/00

(54) **WEICHMACHER**
PLASTICIZER
PLASTIFIANT

(30) Priorität: 21.12.2022 EP 22215338
(43) Veröffentlichungstag der Anmeldung: 29.10.2025
(73) Patentinhaber: BASF SE, 67056 Ludwigshafen am Rhein (DE)
(72) Erfinder: GRIMM, Axel, 67056 Ludwigshafen am Rhein (DE); KASCHEL, Johannes, 67056 Ludwigshafen am Rhein (DE); WITZEL, Sina, 67056 Ludwigshafen am Rhein (DE); MORGENSTERN, Herbert, 67056 Ludwigshafen am Rhein (DE); PFEIFFER, Matthias, 67056 Ludwigshafen am Rhein (DE)
(74) Vertreter: BASF IP Association
(86) Internationale Anmeldenummer: PCT/EP2023/086780
(87) Internationale Veröffentlichungsnummer: WO 2024/133359

(56) Entgegenhaltungen:
- EP-A2- 0 366 089
- US-A- 2 665 303

## Beschreibung

Die vorliegende Erfindung betrifft bestimmte Dicarbonsäurediester und ihre Verwendung als Weichmacher, eine Weichmacher-Zusammensetzung, sowie eine Formmasse oder ein Plastisol enthaltend die Dicarbonsäurediester.

Weichmacher werden in Polymere oder Elastomere eingearbeitet, um deren Flexibilität oder Verarbeitbarkeit zu erhöhen. Am häufigsten werden Weichmacher bei der Herstellung von "weichgemachten" oder flexiblen Polyvinylchlorid (PVC)-Produkten verwendet. Weichmacher lassen sich anhand ihrer chemischen Struktur charakterisieren. Die wichtigste chemische Klasse von Weichmachern sind die Ester aliphatischer oder aromatischer Polycarbonsäuren. Unter den am häufigsten verwendeten aliphatischen Dicarbonsäuren ist z.B. die Adipinsäure, die nach Veresterung mit Alkoholkomponenten zu Adipinsäureestern (Adipaten) als Weichmacher für Polymere, z.B. für Thermoplasten, verwendet wird.

Weichmacher mit einer geringen Flüchtigkeit bei gleichzeitig einer sehr niedrigen Kältebruchtemperatur werden hauptsächlich im Automobilbereich eingesetzt. Vorzugsweise handelt es sich dabei um lineare Phthalsäurediester mit linearen C₉ bis C₁₃-Alkoholen. So hat z.B. ein Mischester aus linearen C₉ bis C₁₃-Alkoholen mit Phthalsäure eine Flüchtigkeit von 0,6% Gewichtsverlust (130 °C/24 h) und eine Kältebruchtemperatur von -45 °C. Niedrige Kältebruchtemperaturen werden z.B. auch mit Adipinsäureestern erreicht. So hat Di-2-Ethylhexyladipat eine Kältebruchtemperatur von unter -50 °C, jedoch bei recht hohen Flüchtigkeitswerten von mehr als 5% Gewichtsverlust (bei 130 °C/24 h). Geringe bis sehr geringe Flüchtigkeiten erreicht man beispielsweise mit Polymer-Weichmachern, jedoch bei nur mäßig guten Kältebruchtemperaturen. So hat z.B. ein Polyadipat aus Neopentylglykol, 1,4-Butandiol mit Isononanol als Endverkappung eine Kältebruchtemperatur von -21 °C und eine Flüchtigkeit bei 130 °C/24 h von 0,5% Gewichtsverlust.

Die Synthese und Verwendung bestimmter 4-Oxoheptandisäureester als Weichmacher ist in US 2,665,303 beschrieben. Darin wird beschrieben, dass es sich bei den C₄ bis C₁₂-Diestern um niedrigviskose bis niedrig-schmelzende Verbindungen handelt. Die Synthese der Diester gelingt durch Direktveresterung der Säure oder durch Umsetzung der Dilactone mit den entsprechenden Alkoholen. Die so erhaltenen Ester zeichnen sich durch hohe Weichmachereffizienz und gute Kältebrucheigenschaften aus. Zu den sonstigen Eigenschaften der entsprechenden Weich-PVC-Massen wird keine Aussage gemacht.

In Gavat et al., Revista de Chimie-Romania 1955, 6, 516-520 werden verschiedene Synthesewege zu Estern der 4-Oxopimelinsäure ausgehend von Furfurylalkoholen beschrieben. Eine Verwendung als Weichmacher in PVC wird erwähnt, jedoch sind keine Daten dazu offenbart.

Moshkin, in: Voprosy Ispol'zovan. Pentozansoderzhashchego Syr'ya, Trudy Vsesoyuz. Soveshchaniya, Riga 1958, 225-254 beschreibt die Synthese von 4-Oxopimelinsäureester aus Furfurylakohol. Die Mitverwendung des Di-2-Ethylhexylesters in PVC wird beschrieben.

Generell unterliegen die bekannten Weichmacher ständigem Optimierungsbedarf, z.B. hinsichtlich deren Flüchtigkeit, Kältebruchtemperatur, Verträglichkeit und/oder toxikologischer Unbedenklichkeit.

Der vorliegenden Erfindung lag daher die Aufgabe zugrunde, Nicht-Phthalat-Weichmacher mit geringer Flüchtigkeit, sehr niedriger Kältebruchtemperatur und guter Verträglichkeit bereitzustellen.

Die Aufgabe wurde gelöst durch Verbindungen der allgemeinen Formel (I) oder Gemische von Verbindungen der allgemeinen Formel (I) 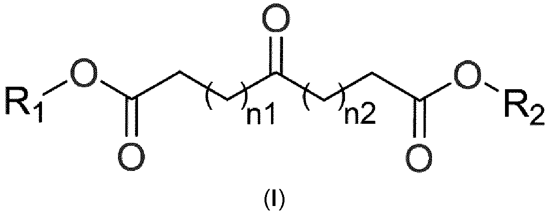 wobei
R₁ und R₂ unabhängig voneinander ausgewählt sind unter C₁₀-C₁₃-Alkyl, wobei zumindest ein Teil der Reste R₁ und/oder R₂ verzweigt ist, und n1 und n2 unabhängig voneinander für 1, 2 oder 3 stehen.

Vorzugsweise sind zumindest 70 Gew.-%, stärker bevorzugt 90 Gew.-% von R₁ und R₂ verzweigt.

Die Verbindungen der allgemeinen Formel (I) können als Reinstoffe vorliegen, d.h. aus identischen Molekülen bestehen. In den Reinstoffen der Verbindungen der allgemeinen Formel (I) steht zumindest einer der Reste R₁ und R₂ für verzweigtes C₁₀-C₁₃-Alkyl, vorzugsweise R₁ und R₂.

Die Verbindungen der allgemeinen Formel (I) können auch als Gemisch von Verbindungen vorliegen, wobei zumindest in einem Teil der Moleküle des Gemisches zumindest einer der Reste R₁ und R₂ verzweigt ist. Das heißt, die Gemische können auch untergeordnete Mengen an Verbindungen der allgemeinen Formel (I) enthalten, in denen R₁ und R₂ geradkettig sind. Solche Gemische werden z.B. durch Veresterung technischer Isoalkohole, die geringe Anteile an linearen Alkoholen enthalten, erhalten.

Im Rahmen der vorliegenden Offenbarung steht die Abkürzung phr (parts per hundred resin) für Gewichtsanteile pro hundert Gewichtsanteile Polymer.

Die Angabe Gewichtsprozent bezieht sich, wenn nichts Gegenteiliges angegeben ist, auf die jeweilige Gesamtmasse.

Eine Mischung ist eine beliebige Mischung aus zwei oder mehr Komponenten, zum Beispiel kann eine Mischung zwei bis fünf oder mehr Komponenten enthalten. Eine Mischung kann auch eine beliebig große Anzahl Komponenten enthalten.

In einer bevorzugten Ausführungsform der Erfindung stehen n1 und n2 für 1. In diesem Fall sind die Verbindungen der allgemeinen Formel (I) Diester der 4-Oxoheptandisäure.

In der Verbindung der allgemeinen Formel (I) können R₁ und R₂ unabhängig voneinander ausgewählt sein unter 2-Propylheptyl, 3,7-dimethyloctyl, iso-Decyl, iso-Undecyl, iso-Dodecyl, und iso-Tridecyl.

Vorzugsweise sind R₁ und R₂ unabhängig voneinander ausgewählt unter 2-Propylheptyl, 3,7-dimethyloctyl, und iso-Tridecyl.

Üblicherweise werden die den genannten iso-Resten, z.B. iso-Octyl, iso-Nonyl, iso-Decyl, iso-Undecyl, iso-Dodecyl, zugrundeliegenden Alkohole nicht als definierte Einzelverbindungen, sondern als Gemische erhalten. Im Rahmen der vorliegenden Erfindung bezeichnet der Begriff "iso-Alkyl" daher sowohl einen verzweigten Alkyl-Rest als auch ein Gemisch eines verzweigten Alkyl-Restes mit wenigstens einem konstitutionsisomeren Alkyl-Rest mit identischer Kohlenstoffzahl.

Auch wenn R₁ und R₂ in einer Verbindung der allgemeinen Formel (I) generell unabhängig voneinander sind, sind R₁ und R₂ bevorzugt gleich oder konstitutionsisomer.

Zum Beispiel kann eine Verbindung der allgemeinen Formel (I) sein:
- I.1 Di-(2-propylheptyl)-4-oxoheptanoat
- I.2 Di-(3,7-dimethyloctyl)-4-oxoheptanoat
- I.3 Di-(iso-decyl)-4-oxoheptanoat
- I.4 Di-(iso-undecyl)-4-oxoheptanoat
- I.5 Di-(iso-dodecyl)-4-oxoheptanoat
- I.6 Di-(iso-tridecyl)-4-oxoheptanoat

Verwendung einer Verbindung der allgemeinen Formel (I) als Weichmacher

In einer Ausführungsform wird die Verbindung der allgemeinen Formel (I) oder das Gemisch von Verbindungen der allgemeinen Formel (I) mit unabhängig voneinander ausgewählten R₁ und R₂ unter C₁₀-C₁₃-Alkyl, wobei zumindest ein Teil der Reste R₁ und/oder R₂ verzweigt ist, und unabhängig voneinander für 1, 2 oder 3 stehenden n1 und n2 als Weichmacher, bevorzugt in einer Formmasse oder einem Plastisol, verwendet.

### Weichmacher-Zusammensetzung

Ein weiterer Gegenstand der vorliegenden Erfindung ist eine Weichmacher-Zusammensetzung, die mindestens eine Verbindung der allgemeinen Formel (I) wie oben beschrieben und mindestens einen weiteren Weichmacher, der von den Verbindungen der allgemeinen Formel (I) unterschiedlich ist, enthält.

Die Weichmacher-Zusammensetzung kann demnach auch eine Mischung von Verbindungen der allgemeinen Formel (I) enthalten, beispielsweise eine Mischung aus Verbindungen der allgemeinen Formel (I), ausgewählt unter I.1, I.2, I.3, I.4, I.5, und I.6.

Der Gehalt der mindestens einen Verbindung der allgemeinen Formel (I) in der Weichmacher-Zusammensetzung beträgt in der Regel wenigstens 10 Gew.-%, bevorzugt 30 bis 90 Gew.-%, stärker bevorzugt 50 bis 80 Gew.-%, und den weiteren Weichmacher in einer Menge von 0 bis 90 Gew.-%, bevorzugt 10 bis 70 Gew.-%, stärker bevorzugt 20 bis 50 Gew.-%, jeweils bezogen auf die Gesamtmasse der Weichmacher-Zusammensetzung. Der Gehalt der mindestens einen Verbindung der allgemeinen Formel (I) in der Weichmacher-Zusammensetzung kann beispielsweise 52, 55, 58, 60, 62, 65, 68, 70, 72, 75, 78 oder 80 Gew.-% betragen.

Die Weichmacher-Zusammensetzung enthält neben der Verbindung der allgemeinen Formel (I) zusätzlich mindestens einen weiteren Weichmacher. Der weitere Weichmacher ist von den Verbindungen der allgemeinen Formel (I) verschieden. Beispielsweise kann der weitere Weichmacher ausgewählt sein unter
- Phthalsäuredialkylestern, z.B. mit 9 bis 13 C-Atomen in den Alkylketten,
- Trimellitsäuretrialkylestern,
- Terephthalsäuredialkylestern, z.B. mit 4 bis 12 C-Atomen in den Alkylketten,
- Benzoesäurealkylestern,
- Dibenzoesäureestern, z.B. Dibenzoesäureestern von Glykolen,
- Hydroxybenzoesäureestern,
- Estern von gesättigten Monocarbonsäuren,
- Estern von ungesättigten Monocarbonsäuren,
- Estern von Hydroxymonocarbonsäuren,
- Estern von Dicarbonsäuren,
- Estern von gesättigten Hydroxydicarbonsäuren,
- Amiden und Estern von aromatischen Sulfonsäuren,
- Pentaerythritolestern,
- Alkylsulfonsäureestern,
- Glycerinestern,
- Isosorbidestern,
- Phosphorsäureestern,
- Citronensäurediestern und Citronensäuretriestern, z.B. acylierten Citronensäuretriestern
- Alkylpyrrolidonderivaten,
- 2,5-Furandicarbonsäureestern, z.B. 2,5-Furandicarbonsäuredialkylestern
- 2,5-Tetrahydrofurandicarbonsäureestern, z.B. 2,5-Tetrahydrofurandicarbonsäuredialkylestern,
- epoxidierten Pflanzenölen,
- epoxidierten Fettsäuremonoalkylestern,
- 1,2-Cyclohexandicarbonsäuredialkylestern, z.B. mit 4 bis 13 C-Atomen in den Alkylketten,
- 1,3-Cyclohexandicarbonsäuredialkylestern, z.B. mit 4 bis 13 C-Atomen in den Alkylketten,
- 1,4-Cyclohexandicarbonsäuredialkylestern, z.B. mit 4 bis 13 C-Atomen in den Alkylketten,
- Polyestern aus aliphatischen und/oder aromatischen Polycarbonsäuren mit wenigstens zweiwertigen Alkoholen,
- weiteren Weichmachern, und
- Mischungen davon.

Ein Phthalsäuredialkylester kann 9 bis 13 C-Atome in den Alkylketten aufweisen. Die Alkylketten können unabhängig voneinander eine unterschiedliche Anzahl von C-Atomen aufweisen. Ein Phthalsäuredialkylester kann beispielsweise Di-isononylphthalat sein.

Ein Trimellitsäuretrialkylester kann 4 bis 13 C-Atome in den Alkylketten aufweisen. Die Alkylketten des Trimellitsäuretrialkylesters können unabhängig voneinander eine unterschiedliche Anzahl an C-Atomen aufweisen.

Ein Benzoesäurealkylester kann 10 bis 13 C-Atome in der Alkylkette aufweisen. Ein Benzoesäurealkylester kann beispielsweise Isodecylbenzoat oder 2-Propylheptylbenzoat sein.

Ein Dibenzoesäureester kann beispielsweise Diethylenglycoldibenzoat, Dipropylenglycoldibenzoat, Tripropylenglycoldibenzoat, oder Dibutylenglycoldibenzoat sein.

Ein gesättigter Monocarbonsäureester kann beispielsweise ein Ester der Essigsäure, ein Ester der Buttersäure, ein Ester der Valeriansäure, oder ein Ester der Milchsäure sein. Ein gesättigter Monocarbonsäureester kann auch ein Ester einer Monocarbonsäure mit einem mehrwertigen Alkohol sein. So kann beispielsweise Valeriansäure mit Pentaerythrit verestert sein.

Ein ungesättigter Monocarbonsäureester kann beispielsweise ein Ester der Acrylsäure sein.

Ein ungesättigter Dicarbonsäurediester kann beispielsweise ein Ester der Maleinsäure sein.

Ein Alkylsulfonsäureester kann 8 bis 22 C-Atome in der Alkylkette aufweisen. Ein Alkylsulfonsäureester kann beispielsweise ein Phenyl- oder Cresylester der Pentadecylsulfonsäure sein.

Ein Isosorbidester ist in der Regel ein Isosorbiddiester, der mit C₈ bis C₁₃-Carbonsäuren verestert ist. Ein Isosorbiddiester kann unterschiedliche oder identische C₈ bis C₁₃-Alkylketten aufweisen.

Ein Phosphorsäureester kann Tri-2-ethylhexylphosphat, Trioctylphosphat, Triphenylphosphat, Isodecyldiphenylphosphat, oder Bis-2(2-ethylhexyl)phenyl-phosphat, 2-Ethylhexyldiphenylphosphat sein.

In einem Citronensäuretriester kann die OH-Gruppe in freier oder carboxylierter Form, beispielsweise acetylierter Form vorliegen. Die Alkylketten des Citronensäuretriesters oder des acetylierten Citronensäuretriesters umfassen unabhängig voneinander 4 bis 8 C-Atome.

Ein Alkylpyrrolidonderivat kann 4 bis 18 C-Atome in der Alkylkette aufweisen.

Ein 2,5-Furandicarbonsäuredialkylester kann 5 bis 13 C-Atome in den Alkylketten aufweisen. Die Alkylketten des 2,5-Furandicarbonsäuredialkylesters können unabhängig voneinander eine unterschiedliche Anzahl an C-Atomen aufweisen.

Ein 2,5-Tetrahydrofurandicarbonsäuredialkylester kann 5 bis 13 C-Atome in den Alkylketten aufweisen. Die Alkylketten des 2,5-Tetrahydrofurandicarbonsäuredialkylesters können unabhängig voneinander eine unterschiedliche Anzahl an C-Atomen aufweisen.

Ein Cyclohexan-1,2-dicarbonsäuredialkylester weist in der Regel 4 bis 13 C-Atome in den Alkylketten auf. Die Alkylketten des Cyclohexan-1,2-dicarbonsäuredialkylesters können unabhängig voneinander eine unterschiedliche Anzahl an C-Atomen aufweisen. Ein Cyclohexan-1,2-dicarbonsäuredialkylester kann Di-(2-ethylhexyl)-1,2-cyclohexansäure-dicarboxylat, Di-(isononyl)-1,2-cyclohexansäuredicarboxylat oder Di-(2-propylheptyl)-1,2-dicarbonsäuredicarboxylat sein.

Ein Cyclohexan-1,3-dicarbonsäuredialkylester kann 4 bis 13 C-Atome in den Alkylketten aufweisen. Die Alkylketten des Cyclohexan-1,3-dicarbonsäuredialkylesters können unabhängig voneinander eine unterschiedliche Anzahl an C-Atomen aufweisen.

Ein Cyclohexan-1,4-dicarbonsäuredialkylester kann 4 bis 13 C-Atome in den Alkylketten aufweisen. Die Alkylketten des Cyclohexan-1,4-dicarbonsäuredialkylesters können unabhängig voneinander eine unterschiedliche Anzahl an C-Atomen aufweisen. Ein Cyclohexan-1,4-dicarbonsäuredialkylester kann beispielsweise Di-(2-ethylhexyl)-cyclohexan-1,4-dicarboxylat, Di-(isononyl)-1,4-cyclohexansäuredicarboxylat oder Di-(2-propylheptyl)-1,4-dicarbonsäuredicarboxylat sein.

Ein Polyester mit aromatischen oder aliphatischen Polycarbonsäuren kann ein Polyester auf Basis der Adipinsäure mit mehrwertigen Alkoholen, wie Dialkylenglykolpolyadipate mit 2 bis 6 C-Atomen in der Alkyleneinheit sein. Beispiele können Polyesteradipate, Polyglykoladipate und Polyesterphthalate sein.

### Polymere

Zweckmäßigerweise wird eine Verbindung der allgemeinen Formel (I) oder Gemisch von Verbindungen der allgemeinen Formel (I) oder eine diese Verbindung oder dieses Gemisch enthaltende Weichmacher-Zusammensetzung (im Folgenden kollektiv: "Weichmacher") als Weichmacher für ein Polymer oder eine Mischung von Polymeren verwendet.

Ein Polymer ist ein Kunststoff. Ein Polymer kann ein Thermoplast oder ein Elastomer sein.

Ein Thermoplast kann in der Regel thermoplastisch verarbeitet werden.

Ein Elastomer kann beispielsweise ein Kautschuk sein. Ein Kautschuk kann ein natürlicher Kautschuk oder ein auf synthetischem Weg hergestellter Kautschuk sein. Auf synthetischem Weg hergestellter Kautschuk kann beispielsweise Polyisopren-Kautschuk, Styrol-Butadien-Kautschuk, Butadien-Kautschuk, Nitril-Butadien-Kautschuk, Chloropren-Kautschuk, und Mischungen davon sein.

Der Weichmacher kann also als Weichmacher für einen Thermoplast oder eine Mischung von Thermoplasten verwendet werden. Der Weichmacher kann auch als Weichmacher für ein Elastomer oder eine Mischung von Elastomeren verwendet werden. Der Weichmacher kann auch als Weichmacher für eine Mischung enthaltend mindestens ein Elastomer und mindestens einen Thermoplasten verwendet werden.

Meist wird der Weichmacher als Weichmacher für Polyvinylchlorid, ein Polyvinylchlorid-Copolymer, eine Mischung von Polymeren, die Polyvinylchlorid enthält, oder ein Plastisol, das vorzugsweise Polyvinylchlorid enthält, verwendet.

Ein Thermoplast kann beispielsweise sein:
- TP.1: ein Homo- oder Copolymer, das in einpolymerisierter Form mindestens ein Monomer enthält, das ausgewählt ist unter C₂ bis C₁₀-Monoolefinen, beispielsweise Ethylen, Propylen, 1,3-Butadien, 2-Chlor-1,3-butadien, Vinylalkoholen oder deren C₂ bis C₄₀-Alkylestern, Vinylacetat, Vinylchlorid, Vinylidenchlorid, Vinylidenfluorid, Tetrafluorethylen, Glycidylacrylat, Glycidylmethacrylat, Acrylaten oder Methacrylaten mit Alkoholkomponenten von verzweigten oder unverzweigten C₁ bis C₁₀-Alkoholen, Vinylaromaten wie Styrol, (Meth)acrylnitril, α,β-ethylenisch ungesättigten Mono- oder Dicarbonsäuren und Maleinsäureanhydrid.
- TP.2: ein Polyvinylester
- TP.3: ein Polycarbonat
- TP.4: ein Polyether
- TP.5: ein Polyetherketon
- TP.6: ein thermoplastisches Polyurethan
- TP.7: ein Polysulfid
- TP.8: ein Polysulfon
- TP.9: ein Polyester
- TP.10: ein Polyalkylenterephthalat
- TP.11: ein Polyhydroxyalkanoat
- TP.12: ein Polybutylensuccinat
- TP.13: ein Polybutylensuccinatadipat
- TP.14: ein Polyacrylat mit gleichen oder unterschiedlichen Alkoholresten aus der Gruppe der C₄ bis C₈-Alkohole wie Butanol, Hexanol, Octanol, 2-Ethylhexanol
- TP.15: ein Polymethylmethacrylat
- TP.16: ein Methylmethacrylat-Butylacrylat-Copolymer
- TP.17: ein Acrylnitril-Butadien-Styrol-Copolymer
- TP.18: ein Ethylen-Propylen-Copolymer
- TP.19: ein Ethylen-Propylen-Dien-Copolymer
- TP.20: ein Polystyrol
- TP.21: ein Styrol-Acrylnitril-Copolymer
- TP.22: ein Acrylnitril-Styrol-Acrylat
- TP.23: ein Styrol-Butadien-Methylmethacrylat-Copolymer
- TP.24: ein Styrol-Maleinsäureanhydrid-Copolymer
- TP.25: ein Styrol-Methacrylsäure-Copolymer
- TP.26: ein Polyoxymethylen
- TP.27: ein Polyvinylalkohol
- TP.28: ein Polyvinylacetat
- TP.29: ein Polyvinylbutyral
- TP.30: ein Polyvinylchlorid
- TP.31: ein Polycaprolacton
- TP.32: Polyhydroxybuttersäure
- TP.33: Polyhydroxyvaleriansäure
- TP.34: Polymilchsäure
- TP.35: Ethylcellulose
- TP.36: Celluloseacetat
- TP.37: Cellulosepropionat
- TP.38: Cellulose-Acetat/Butyrat

Im Allgemeinen wird Polyvinylchlorid durch Homopolymerisation von Vinylchlorid erhalten. Polyvinylchlorid kann beispielsweise durch Suspensionspolymerisation, wie Mikrosuspensionspolymerisation, oder Massenpolymerisation hergestellt werden. Die Herstellung von Polyvinylchlorid durch Polymerisation von Vinylchlorid sowie Herstellung und Zusammensetzung von weichgemachten Polyvinylchlorid sind beispielsweise beschrieben in "Becker/Braun, Kunststoff-Handbuch, Band 2/1: Polyvinylchlorid", 2. Auflage, Carl Hanser Verlag, München.

Der die Molmasse des Polyvinylchlorids charakterisierende K-Wert wird nach DIN-EN 1628-2 (Nov 1999) bestimmt und liegt für das mit dem Weichmacher weichgemachte Polyvinylchlorid meist im Bereich von 57 bis 90, bevorzugt 61 bis 85, besonders bevorzugt 64 bis 80.

Vorteilhafterweise zeichnet sich der vorliegende Weichmacher durch eine hohe Verträglichkeit mit dem weichzumachenden Kunststoff aus. Zudem kann durch den vorliegenden Weichmacher das Gelierverhalten der weichgemachten Kunststoffe positiv beeinflusst werden. Weiterhin kann sich der vorliegende Weichmacher durch eine geringe Flüchtigkeit auszeichnen, sowohl bei der Verarbeitung als auch während des Gebrauchs der Endprodukte. Ebenfalls kann der Weichmacher einen vorteilhaften Effekt auf die mechanischen Eigenschaften der damit weichgemachten Kunststoffe haben.

Gute mechanische Eigenschaften können sich beispielsweise in einer hohen Elastizität der weichgemachten Kunststoffe widerspiegeln. Ein Maß für die Elastizität von weichgemachten Kunststoffen ist die Shore A-Härte. Je niedriger die Shore A-Härte, desto höher die Elastizität der weichgemachten Kunststoffe.

Ein Maß für gute Geliereigenschaften kann eine niedrige Lösetemperatur/Geliertemperatur sein.

Die Verträglichkeit (Permanenz) von Weichmachern in weichgemachten Kunststoffen charakterisiert, in welchem Ausmaß Weichmacher während des Gebrauchs der weichgemachten Kunststoffe zum Ausschwitzen neigen und dadurch die Gebrauchseigenschaften der Kunststoffe beeinträchtigt werden.

Eine geringe Flüchtigkeit bei der Verarbeitung kann beispielsweise durch eine geringe Prozessflüchtigkeit widergespiegelt werden.

Eine geringe Flüchtigkeit beim Gebrauch des Endprodukts kann beispielsweise durch eine geringe Folienflüchtigkeit widergespiegelt werden.

### Formmasse und Plastisol

Ein weiterer Gegenstand der vorliegenden Erfindung ist eine Formmasse oder ein Plastisol, wobei die Formmasse oder das Plastisol mindestens eine Verbindung der allgemeinen Formel (I) oder eine Weichmacher-Zusammensetzung wie oben beschrieben, und mindestens ein Polymer enthält.

Der Weichmacher kann also als Weichmacher in einer Formmasse oder einem Plastisol verwendet werden.

Im Allgemeinen versteht man unter dem Begriff "Formmasse" un- oder vorgeformte Stoffe, die mittels mechanischer Krafteinwirkung und erhöhten Temperaturen durch eine spanlose Formgebung zu Halbzeugen oder Fertigteilen verarbeitet werden.

Im Allgemeinen handelt es sich bei einem Plastisol um eine Suspension von feinpulvrigem Polymer in flüssigem Weichmacher, wobei die Lösegeschwindigkeit des Polymers im flüssigen Weichmacher bei Raumtemperatur sehr gering ist. Beim Erwärmen der Suspension von feinpulvrigem Polymer in flüssigem Weichmacher bildet sich eine weitgehend homogene Phase zwischen Polymer und Weichmacher. Dabei quellen und verbinden sich (gelieren) die einzelnen isolierten Kunststoffaggregate zu einem dreidimensionalen hochviskosen Gel. Dieser Vorgang wird in der Regel als Gelieren bezeichnet und findet ab einer gewissen Mindesttemperatur statt. Diese Mindesttemperatur wird generell als Gelier- oder Lösetemperatur bezeichnet. Das Einbringen der dafür nötigen Wärme kann über die Parameter Temperatur und/oder Verweilzeit erfolgen. Je schneller die Gelierung abläuft, desto geringer kann die Temperatur (bei gleicher Verweilzeit) oder die Verweilzeit (bei gleicher Temperatur) gewählt werden. Ein Indiz für die Geschwindigkeit der Gelierung ist die Lösetemperatur, d.h. je niedriger diese ist, desto schneller geliert das Plastisol.

Die Formmasse oder das Plastisol kann auch eine Mischung von Polymeren enthalten.

In einer Ausführungsform der Erfindung ist das Polymer ausgewählt unter einem Thermoplast, einem Elastomer, und Mischungen davon.

Meist ist in der den Weichmacher enthaltenden Formmasse oder dem den Weichmacher enthaltenden Plastisol mindestens ein Thermoplast enthalten. Die Formmasse oder das Plastisol kann auch eine Mischung von Thermoplasten enthalten.

In einer Ausführungsform ist der Thermoplast ausgewählt unter
- Homo- oder Copolymeren, die mindestens ein Monomer in einpolymerisierter Form enthalten, ausgewählt unter C₂-C₁₀-Monoolefinen wie Ethylen oder Propylen, 1,3-Butadien, 2-Chlor-1,3-Butadien, Vinylalkoholen und deren C₂-C₁₀-Alkylestern, Vinylacetat, Vinylchlorid, Vinylidenchlorid, Vinylidenfluorid, Tetrafluorethylen, Glycidylacrylat, Glycidylmethacrylat, Acrylaten und Methacrylaten von C₁-C₁₀-Alkoholen, Vinylaromaten wie Styrol, Acrylnitril, Methacrylnitril, α,β-ethylenisch ungesättigte Mono- oder Dicarbonsäuren und Maleinsäureanhydrid,
- Homo- oder Copolymeren von Vinylacetalen, Polyvinylestern, Polycarbonaten, Polyestern, Polyethern, Polyetherketonen, thermoplastischen Polyurethanen, Polysulfiden, Polysulfonen, Polyethersulfonen, Polyacrylaten, Polymethylmethacrylaten, Polystyrolen, Polyvinylalkoholen, Polyvinylacetaten, Polyvinylbutyralen, Polyvinylchloriden, Polycaprolactonen, Cellulosealkylestern und Mischungen davon,
und
das Elastomer ausgewählt unter natürlichem Kautschuk, und synthetischem Kautschuk wie Polyisopren-Kautschuk, Styrol-Butadien-Kautschuk, Butadien-Kautschuk, Nitril-Butadien-Kautschuk, Chloropren-Kautschuk und Mischungen davon.

Die Formmasse oder das Plastisol kann beispielsweise wie in Tabelle 1 gezeigt zusammengesetzt sein.

**Tabelle 1.**

| Thermoplast | I.1 | I.2 | I.6 |
|---|---|---|---|
| TP 30 | X | | |
| TP 30 | | X | |
| TP 30 | | | X |
| TP 29 | X | | |
| TP 29 | | X | |
| TP 29 | | | X |
| Homo- und/oder Copolymere von Vinylacetat | X | | |
| Homo- und/oder Copolymere von Vinylacetat | | X | |
| Homo- und/oder Copolymere von Vinylacetat | | | X |
| Homo- und/oder Copolymere von Styrol | X | | |
| Homo- und/oder Copolymere von Styrol | | X | |
| Homo- und/oder Copolymere von Styrol | | | X |
| TP 14 | X | | |
| TP 14 | | X | |
| TP 14 | | | X |
| TP 6 | X | | |
| TP 6 | | X | |
| TP 6 | | | X |
| TP 7 | X | | |
| TP 7 | | X | |
| TP 7 | | | X |

Abhängig vom Polymer, das in der Formmasse enthalten ist, kann es sein, dass zur Erzielung der gewünschten thermoplastischen Eigenschaften unterschiedliche Mengen des Weichmachers in der Formmasse enthalten sein müssen. Die Einstellung der gewünschten thermoplastischen Eigenschaften der Formmasse unterliegt generell der Routinetätigkeit des Fachmanns.

Ist kein Polyvinylchlorid in der Formmasse enthalten, beträgt die Menge an Weichmacher in der Formmasse in der Regel 0.5 bis 300 phr. Es kann bevorzugt sein, dass die Menge des Weichmachers in der Formmasse 1.0 bis 130 phr beträgt. Es kann stärker bevorzugt sein, dass die Menge des Weichmachers in der Formmasse 2.0 bis 100 phr beträgt. Die Menge des Weichmachers, die in der Formmasse enthalten ist, kann beispielsweise 5, 10, 15, 20, 25, 30, 35, 40, 45, 50, 55, 60, 65, 70, 75, 80, 85, 90 oder 95 phr betragen.

Ist Polyvinylchlorid in der Formmasse enthalten, beträgt die Menge des Weichmachers in der Formmasse in der Regel 5 bis 300 phr. Es kann bevorzugt sein, dass die Menge des Weichmachers in der Formmasse 15 bis 200 phr beträgt. Es kann stärker bevorzugt sein, dass die Menge des Weichmachers in der Formmasse 30 bis 150 phr beträgt. Die Menge des Weichmachers, die in der Formmasse enthalten ist, kann beispielsweise 35, 40, 45, 50, 55, 60, 65, 70, 75, 80, 85, 90, 95, 100, 105, 110, 115, 120, 125, 130, 135, 140, oder 145 phr betragen.

In der Regel enthält die Formmasse 20 bis 90 Gew.-%, bevorzugt 40 bis 90 Gew.-%, stärker bevorzugt 45 bis 85 Gew.-% Polyvinylchlorid. Beispielsweise kann die Formmasse 50, 55, 60, 65, 70, 75 oder 80 Gew.-% Polyvinylchlorid enthalten.

Abhängig von dem Polymer, das in dem Plastisol enthalten ist, kann es sein, dass zur Erzielung der gewünschten Plastisol-Eigenschaften unterschiedliche Mengen des Weichmachers im Plastisol enthalten sein müssen. Die Einstellung der gewünschten Plastisol-Eigenschaften unterliegt generell der Routinetätigkeit des Fachmanns.

Enthält das Plastisol Polyvinylchlorid, beträgt der Anteil an Weichmacher im Plastisol üblicherweise 30 bis 400 phr, bevorzugt 50 bis 200 phr. Der Gehalt an Weichmachern der allgemeinen Formel (I) in einem Polyvinylchlorid enthaltenden Plastisol beträgt meist mindestens 10 phr, bevorzugt mindestens 15 phr und stärker bevorzugt mindestens 20 phr.

### Zusatzstoffe Formmasse oder Plastisol mit Thermoplasten

Zweckmäßigerweise kann die mindestens einen Thermoplasten und den Weichmacher enthaltende Formmasse oder das mindestens einen Thermoplasten und den Weichmacher enthaltende Plastisol zusätzlich mindestens einen Zusatzstoff enthalten. Der Zusatzstoff kann ausgewählt sein unter Stabilisatoren, Gleitmitteln, Füllstoffen, Farbmitteln, Flamminhibitoren, Lichtstabilisatoren, Treibmitteln, polymere Verarbeitungsmitteln, Schlagzähverbesserern, optischen Aufhellern, Antistatika, Biostabilisatoren, und Mischungen davon.

Die im Folgenden beschriebenen Zusatzstoffe stellen keine Einschränkung der Formmasse oder des Plastisols dar, sondern dienen lediglich der Erläuterung der Formmasse oder des Plastisols.

Stabilisatoren können die üblichen Polyvinylchlorid-Stabilisatoren in fester und flüssiger Form sein, wie z.B. Ca/Zn-, Ba/Zn-, Pb-, Sn-Stabilisatoren, säurebindende Schichtsilikate, Carbonate wie Hydrotalcit oder Mischungen davon.

Die Formmasse oder das Plastisol kann einen Gehalt an Stabilisatoren von 0.05 bis 7 Gew.-%, bevorzugt 0.1 bis 5 Gew.-%, stärker bevorzugt 0.5 bis 3 Gew.-% aufweisen, bezogen auf das Gesamtgewicht der Formmasse oder des Plastisols.

Gleitmittel dienen in der Regel dazu, die Haftung zwischen der Formmasse oder des Plastisols und Oberflächen zu reduzieren und sollen beispielsweise die Reibungskräfte beim Mischen, Plastifizieren oder Verformen herabsetzen.

Als Gleitmittel in der Formmasse oder im Plastisol können alle in der Kunststoffverarbeitung eingesetzten gängigen Gleitmittel verwendet werden. In der Kunststoffverarbeitung gängige Gleitmittel sind beispielsweise Kohlenwasserstoffe, wie Öle, Paraffine, PE-Wachse oder Mischungen davon, Fettalkohole mit 6 bis 20 C-Atomen, Ketone, Carbonsäuren, wie Fettsäuren, Montansäuren oder Mischungen davon, oxidierte PE-Wachse, Metallsalze von Carbonsäuren, Carbonsäureamide, Carbonsäureester, die aus der Veresterung von Alkoholen wie Ethanol, Fettalkoholen, Glycerin, Ethandiol oder Pentaerythrit mit langkettigen Carbonsäuren hervorgehen.

Die Formmasse oder das Plastisol kann einen Gehalt an Gleitmittel von 0.01 bis 10 Gew.-%, bevorzugt 0.05 bis 5 Gew.-%, stärker bevorzugt 0.2 bis 2 Gew.-% ausweisen, bezogen auf das Gesamtgewicht der Formmasse oder des Plastisols.

Füllstoffe werden im Allgemeinen eingesetzt, um die Druck-, Zug-, und/oder Biegefestigkeit, die Härte und/oder die Wärmeformbeständigkeit der Formmasse oder des Plastisols positiv zu beeinflussen.

Als Füllstoffe können beispielsweise Ruß und/oder anorganische Füllstoffe in der Formmasse oder im Plastisol enthalten sein. Anorganische Füllstoffe können ausgewählt sein unter natürlichen Calciumcarbonaten, wie Kreiden, Kalkstein, Marmoren, synthetischen Calciumcarbonaten, Dolomit, Silikaten, Kieselsäuren, Sand, Diatomeenerden, Aluminiumsilikaten, wie Kaolin, Glimmer, Feldspat, oder Mischungen aus zwei oder mehr der zuvor genannten Füllstoffe.

Die Formmasse oder das Plastisol kann einen Gehalt an Füllstoffen von 0.01 bis 80 Gew.-%, bevorzugt 0.01 bis 60 Gew.-%, stärker bevorzugt 1 bis 40 Gew.-% aufweisen, bezogen auf das Gesamtgewicht der Formmasse oder des Plastisols. So kann die Formmasse oder das Plastisol einen Gehalt an Füllstoffen von 2, 5, 8, 10, 12, 15, 18, 20, 22, 25, 27, 30, 33, 36 oder 39 Gew.-% aufweisen.

Farbmittel können dazu dienen, die Formmasse oder das Plastisol an unterschiedliche Einsatzmöglichkeiten anzupassen. Farbmittel können beispielsweise Pigmente oder Farbstoffe sein.

Als Pigmente können beispielsweise anorganische und/oder organische Pigmente in der Formmasse oder im Plastisol enthalten sein. Anorganische Pigmente können Kobalt-Pigmente wie CoO/Al₂O₃ und/oder Chrom-Pigmente wie Cr₂O₃ sein. Organische Pigmente können Monoazopigmente, kondensierte Azopigmente, Azomethinpigmente, Anthrachinonpigmente, Chinacridone, Phthalocyaninpigmente und/oder Dioxazinpigmente sein.

Die Formmasse oder das Plastisol kann einen Gehalt an Farbmitteln von 0.01 bis 10 Gew.-%, bevorzugt 0.05 bis 5 Gew.-%, stärker bevorzugt 0.1 bis 3 Gew.-% aufweisen, bezogen auf das Gesamtgewicht der Formmasse oder des Plastisols.

Flamminhibitoren können dazu dienen, die Entflammbarkeit der Formmasse oder des Plastisols zu vermindern und die Rauchbildung beim Verbrennen zu reduzieren.

Flamminhibitoren, die in der Formmasse oder im Plastisol enthalten sein können, können beispielsweise Antimontrioxid, Chlorparaffin, Phosphatester, Aluminiumhydroxid und/oder Borverbindungen sein.

Die Formmasse oder das Plastisol kann einen Gehalt an Flamminhibitoren von 0.01 bis 10 Gew.-%, bevorzugt 0.2 bis 5 Gew.-%, stärker bevorzugt 0.5 bis 2 Gew.-% aufweisen, bezogen auf das Gesamtgewicht der Formmasse oder des Plastisols.

Lichtstabilisatoren, wie UV-Absorber, können dazu dienen, die Formmasse oder das Plastisol durch eine Schädigung durch den Einfluss von Licht zu schützen.

Lichtstabilisatoren können beispielsweise Hydroxybenzophenone, Hydroxyphenylbenzotriazole, Cyanoacrylate, "hindered amine light stabilizers" wie Derivate von 2,2,6,6-Tetramethylpiperidin oder Mischungen der zuvor genannten Verbindungen sein.

Die Formmasse oder das Plastisol kann einen Gehalt an Lichtstabilisatoren von 0.01 bis 7 Gew.-%, bevorzugt 0.02 bis 4 Gew.-%, stärker bevorzugt 0.05 bis 3 Gew.-% aufweisen, bezogen auf das Gesamtgewicht der Formmasse oder des Plastisols.

### Zusatzstoffe Formmasse mit Elastomeren

In der Formmasse kann der Weichmacher und mindestens ein Elastomer enthalten sein. In der Formmasse kann auch der Weichmacher und eine Mischung von Elastomeren enthalten sein.

Wie oben beschrieben kann ein Elastomer beispielsweise ein Kautschuk sein. Ein Kautschuk kann ein natürlicher Kautschuk oder ein auf synthetischem Weg hergestellter Kautschuk sein. Auf synthetischem Weg hergestellter Kautschuk kann beispielsweise Polyisopren-Kautschuk, Styrol-Butadien-Kautschuk, Butadien-Kautschuk, Nitril-Butadien-Kautschuk, Chloropren-Kautschuk, und Mischungen davon sein.

In der Regel enthält die Formmasse mindestens natürlichen Kautschuk und/oder mindestens einen synthetischen Kautschuk wobei sich der enthaltene Kautschuk oder die Kautschukmischung mit Schwefel vulkanisieren lassen.

Meist enthält die Formmasse mindestens ein Elastomer mit einem Anteil von 20 bis 95 Gew.-% bezogen auf das Gesamtgewicht der Formmasse. Es kann bevorzugt sein, dass die Formmasse mindestens ein Elastomer mit einem Anteil von 45 bis 90 Gew.-% enthält. Weiter kann es bevorzugt sein, dass die Formmasse mindestens ein Elastomer mit einem Anteil 50 bis 85 Gew.-% enthält. Die Formmasse kann beispielsweise 55, 60, 65, 70, 75 oder 80 Gew.-% mindestens eines Elastomers enthalten.

Ist in der Formmasse mindestens ein Elastomer enthalten, speziell mindestens natürlicher Kautschuk oder mindestens ein synthetischer Kautschuk, beträgt die Menge des Weichmachers in der Formmasse in der Regel 1 bis 60 phr. Es kann bevorzugt sein, dass die Menge des Weichmachers in der Formmasse 2 bis 40 phr und stärker bevorzugt 3 bis 30 phr beträgt. Die Menge des Weichmachers, die in der Formmasse enthalten ist, kann beispielsweise 5, 10, 15, 20 oder 25 phr betragen.

Auch kann in der Formmasse eine Mischung von mindestens einem Thermoplast und mindestens einem Elastomer enthalten sein. So kann in der Formmasse eine Mischung von Polyvinylchlorid und mindestens einem Elastomer enthalten sein.

Ist in der Formmasse Polyvinylchlorid und mindestens ein Elastomer enthalten, beträgt der Gehalt an Elastomer in der Regel 1 bis 50 Gew.-% bezogen auf das Gesamtgewicht der Formmasse. Es kann bevorzugt sein, dass der Gehalt an Elastomer 3 bis 40 Gew.-% bezogen auf das Gesamtgewicht der Formmasse beträgt. Es kann stärker bevorzugt sein, dass der Gehalt an Elastomer 5 bis 30 Gew.-% bezogen auf das Gesamtgewicht der Formmasse beträgt. Die Formmasse kann beispielsweise 10, 15, 20 oder 25 Gew.-% an Elastomer enthalten.

Abhängig von der Zusammensetzung der Mischung von Polyvinylchlorid und mindestens einem Elastomer in der Formmasse kann die benötigte Menge an Weichmacher in der Formmasse zur Erzielung der gewünschten Eigenschaften stark variieren. Es entspricht der Routinetätigkeit des Fachmanns, entsprechende Mengen des Weichmachers zu verwenden, um die gewünschten Eigenschaften zu erzielen.

In der Regel beträgt die Menge des Weichmachers in der Formmasse, die Polyvinylchlorid und mindestens Elastomer enthält 0.5 bis 300 phr. Es kann bevorzugt sein, dass die Menge des Weichmachers in der Formmasse, die Polyvinylchlorid und mindestens ein Elastomer enthält, 1 bis 150 phr und stärker bevorzugt 2 bis 120 phr beträgt. Die Menge des Weichmachers, die in der Formmasse enthalten ist, kann beispielsweise 5, 10, 15, 20, 25, 30, 35, 40 ,45, 50, 55, 60, 65, 70, 75, 80, 85, 90, 95, 100, 105, 110 oder 115 phr betragen.

Zweckmäßigerweise kann die mindestens ein Elastomer und den Weichmacher enthaltende Formmasse zusätzlich mindestens einen Zusatzstoff enthalten. Der Zusatzstoff kann ausgewählt sein unter Ruß, Siliziumdioxid, Phenolharzen, Vulkanisier- oder Vernetzungsmitteln, Vulkanisier- oder Vernetzungsbeschleunigern, Aktivatoren, verschiedenen Ölen, Alterungsschutzmitteln oder Mischungen der genannten Zusatzstoffe.

Weitere Zusatzstoffe können Stoffe sein, die der Fachmann aufgrund seines Fachwissens in Reifen oder andere Kautschukmassen einmischen würde, um einen bestimmten Effekt zu erzielen.

### Verwendung der Formmassen

Die Formmasse kann beispielsweise zur Herstellung von Formkörpern, Handschuhen, Folien, Tapeten, oder heterogenen Fußböden, oder zur Textilbeschichtung verwendet werden.

Formkörper können beispielsweise Behältnisse, Apparate oder geschäumte Vorrichtungen sein.

Behältnisse können beispielsweise Gehäuse von Elektrogeräten, wie Küchengeräte oder Computergehäuse, Rohre, Schläuche, wie Wasser- oder Bewässerungsschläuche, Industrie-Gummischläuche, Chemieschläuche, Ummantelungen für Draht oder Kabel, Ummantelungen für Werkzeuge, Fahrrad-, Roller- oder Schubkarrengriffe, Metallbeschichtungen oder Verpackungsbehälter sein.

Apparate können beispielsweise Werkzeuge, Möbel, wie z.B. Stühle, Regale, Tische, Schallplatten, Profile, wie z.B. Fensterprofile, Bodenprofile für den Außenbereich, oder Profile für Förderbänder, Komponenten für den Fahrzeugbau wie z.B. Karosseriebestandteile, Unterbodenschutz, oder Vibrationsdämpfer, oder Radiergummis sein.

Geschäumte Vorrichtungen können beispielsweise Polster, Matratzen, Schaumstoffe oder Dämmmaterialien sein.

Folien können beispielsweise Planen, wie LKW-Planen, Dachplanen, Geomembranplanen, Stadiondächer oder Zeltplanen, Dichtungen, Verbundfolien, wie Folien für Verbundsicherheitsglas, Selbstklebefolien, Kaschierfolien, Schrumpffolien, Bodenbeläge für den Außenbereich, Klebebandfolien, Beschichtungen, Schwimmteichfolien, Zierteichfolien, Tischdecken oder Kunstleder sein.

Die Formmasse kann zur Herstellung von Formkörpern oder Folien verwendet werden, die direkt in Kontakt mit dem Menschen oder Nahrungsmittel kommen.

Formkörper oder Folien, die direkt in Kontakt mit dem Menschen oder Nahrungsmittel kommen können beispielsweise Medizinprodukte, Hygieneprodukte, Lebensmittelverpackungen, Produkte für den Innenraum, Baby- und Kinderprodukte, Kinderpflegeartikel, Sport- oder Freizeitprodukte, Bekleidung, Fasern oder Gewebe sein.

Medizinprodukte, die unter Verwendung der Formmasse hergestellt werden können, können beispielsweise Schläuche für enterale Ernährung oder Hämodialyse, Beatmungsschläuche, Drainageschläuche, Infusionsschläuche, Infusionsbeutel, Blutbeutel, Katheter, Trachealtuben, Einmalspritzen, Handschuhe oder Atemmasken sein.

Lebensmittelverpackungen, die unter Verwendung der Formmasse hergestellt werden können, können beispielsweise Frischhaltefolien, Lebensmittelschläuche, Trinkwasserschläuche, Behälter zur Aufbewahrung oder zum Einfrieren von Lebensmitteln, Deckeldichtungen, Verschlusskappen, Kronkorken oder künstliche Weinkorken sein.

Produkte für den Innenraum, die unter Verwendung der Formmasse hergestellt werden können, können beispielsweise Bodenbeläge, welche homogen oder aus mehreren Schichten, bestehend aus mindestens einer geschäumten Schicht, aufgebaut sein können, wie Fußbodenbeläge, Schmutzfängermatten, Sportböden, Luxury Vinyl Tiles (LVT), Kunstleder, Wandbeläge, geschäumte oder nicht geschäumte Tapeten in Gebäuden, Verkleidungen oder Konsolenabdeckungen in Fahrzeugen sein.

Baby- und Kinderprodukte, die unter Verwendung der Formmasse hergestellt werden können, können beispielsweise Spielzeug wie Puppen, Spielfiguren oder Kneten, aufblasbares Spielzeug, wie Bälle oder Ringe, Stoppersocken, Schwimmhilfen, Kinderwagenabdeckungen, Wickelauflagen, Wärmflaschen, Beißringe oder Fläschchen sein.

Sport- oder Freizeitprodukte, die unter Verwendung der Formmasse hergestellt werden können, können beispielsweise Gymnastikbälle, Übungsmatten, Sitzkissen, Massagebälle oder -rollen, Schuhe, Schuhsohlen, Bälle, Luftmatratzen, Sicherheitsbrillen, Handschuhe oder Trinkflaschen sein.

Bekleidung, die unter Verwendung der Formmasse hergestellt werden kann, kann beispielsweise Latexkleidung, Schutzbekleidung, Regenjacken oder Gummistiefel sein.

### Verwendung der Plastisole

Plastisole werden üblicherweise bei Umgebungstemperatur durch verschiedene Verfahren, wie Streichverfahren, Gießverfahren, wie das Schalengieß- oder Rotationsgießverfahren, Tauchverfahren, Druckverfahren, wie Siebdruckverfahren, Spritzverfahren und dergleichen in die Form des fertigen Produkts gebracht. Anschließend erfolgt durch Erwärmung die Gelierung, wobei nach Abkühlung ein homogenes, mehr oder weniger flexibles Produkt erhalten wird.

Das Plastisol kann zur Herstellung von Folien, Tapeten, nahtlosen Hohlkörpern, Handschuhen, heterogenen Fußböden oder für Anwendung im Textilbereich, wie z.B. für Textilbeschichtungen verwendet werden.

Folien können beispielsweise LKW-Planen, Dachplanen, Abdeckungen im Allgemeinen, wie Bootsabdeckungen, Kinderwagenabdeckungen oder Stadiondächer, Zeltplanen, Geomembranen, Tischdecken, Beschichtungen, Schwimmteichfolien, Kunstleder oder Zierteichfolien sein.

Handschuhe können beispielsweise, Gartenhandschuhe, medizinische Handschuhe, Chemikalienhandschuhe, Schutzhandschuhe oder Einweghandschuhe sein.

Weiterhin kann das Plastisol zur Herstellung beispielsweise von Dichtungen, Deckeldichtungen, Verkleidungen oder Konsolenabdeckungen in Fahrzeugen, Puppen, Spielfiguren oder Kneten, aufblasbares Spielzeug, wie Bälle oder Ringe, Stoppersocken, Schwimmhilfen, Wickelauflagen, Gymnastikbälle, Übungsmatten, Sitzkissen, Vibratoren, Massagebälle oder -rollen, Latexkleidung, Schutzbekleidung, Regenjacken oder Gummistiefel verwendet werden.

Das Plastisol enthält meist Polyvinylchlorid.

### Nicht-PVC-Anwendungen

Auch Gegenstand der vorliegenden Offenbarung ist die Verwendung des Weichmachers als Kalandrier-Hilfsmittel oder Rheologie-Hilfsmittel. Ebenfalls Gegenstand der vorliegenden Offenbarung ist die Verwendung des Weichmachers in oberflächenaktiven Zusammensetzungen wie Fließ- oder Filmbindehilfen, Entschäumern, Schaumverhütern, Benetzungsmitteln, Koaleszenzmitteln oder Emulgatoren. Der Weichmacher kann auch in Schmierstoffen wie Schmierölen, Schmierfetten oder Schmierpasten verwendet werden. Weiter kann der Weichmacher als Quenchmittel für chemische Reaktionen, Phlegmatisierungsmittel, in Pharmazeutischen Produkten, in Klebstoffen, in Dichtstoffen, in Tinten, wie Druckertinten, in Schlagzähmodifizierern oder Stellmitteln verwendet werden.

### Produkte enthaltend den Weichmacher

Gegenstand der Offenbarung sind Formkörper oder Folien, die den Weichmacher enthalten. Es wird auf die bei der Verwendung von Formmassen zur Herstellung von Formkörpern oder Folien gemachten Angaben zu Formkörpern oder Folien Bezug genommen. Die dabei angeführten Beispiele für Formkörper oder Folien sind zur Auslegung der Begrifflichkeiten Formkörper oder Folie in diesem Abschnitt heranzuziehen.

### Herstellung der Verbindungen der allgemeinen Formel (I)

Zur Herstellung von Verbindungen der allgemeinen Formel (I) können leicht zugängliche Edukte verwendet werden. Ein besonderer ökonomischer und ökologischer Vorteil kann dabei in der Möglichkeit liegen, Verbindungen der allgemeinen Formel (I) aus petrochemischen und/oder nachwachsenden Rohstoffen herzustellen.

Verbindungen der allgemeinen Formel (I) können beispielsweise durch Veresterung von entsprechenden Dicarbonsäuren mit den entsprechenden aliphatischen Alkoholen hergestellt werden. Verfahren und spezifische Verfahrensmaßnahmen sind entweder dem Fachmann bekannt oder erschließen sich ihm durch sein allgemeines Fachwissen.

Dazu zählt die Umsetzung mindestens einer Alkoholkomponente, ausgewählt aus den Alkoholen R₁-OH und R₂-OH mit einer entsprechenden Dicarbonsäure. Geeignete Derivate sind z.B. die Säurehalogenide und Säureanhydride. Ein Säurehalogenid kann beispielsweise ein Säurechlorid sein. Die Umsetzung kann in Gegenwart eines Veresterungskatalysators erfolgen.

Als Veresterungskatalysator können die dafür üblichen Katalysatoren eingesetzt werden, z.B. Mineralsäuren, wie Schwefelsäure oder Phosphorsäure; organische Sulfonsäuren, wie Methansulfonsäure oder p-Toluolsulfonsäure; amphotere Katalysatoren, insbesondere Titan-, Zinn (IV)- oder Zirkoniumverbindungen, wie Tetraalkoxytitane, z.B. Tetrabutoxytitan, oder Zinn-(IV)-oxid. Das bei der Reaktion entstehende Wasser kann durch übliche Maßnahmen, z.B. destillativ, entfernt werden. So beschreibt beispielsweise die WO 02/038531 ein Verfahren zur Herstellung von Estern, bei dem man a) in einer Reaktionszone ein im Wesentlichen aus der Säurekomponente oder einem Anhydrid davon und der Alkoholkomponente bestehendes Gemisch in Gegenwart eines Veresterungskatalysators zum Sieden erhitzt, b) die Alkohol und Wasser enthaltenden Dämpfe rektifikativ in eine alkoholreiche Fraktion und eine wasserreiche Fraktion auftrennt, c) die alkoholreiche Fraktion in die Reaktionszone zurückführt und die wasserreiche Fraktion aus dem Verfahren ausleitet. Als Veresterungskatalysatoren werden die zuvor genannten Katalysatoren eingesetzt. Der Veresterungskatalysator wird in einer wirksamen Menge eingesetzt, die üblicherweise im Bereich von 0,05 bis 10 Gew.-%, vorzugsweise 0,1 bis 5 Gew.-%, bezogen auf die Summe von Säurekomponente (oder Anhydrid) und Alkoholkomponente, liegt. Weitere detaillierte Darstellungen zur Durchführung von Veresterungsverfahren finden sich beispielsweise in der US 6,310,235 B1, US 5,324,853 A, DE-A 2612355 (Derwent Abstract Nr. DW 77-72638 Y) oder DE-A 1945359 (Derwent Abstract Nr. DW 73-27151 U). Auf die genannten Dokumente wird in vollem Umfang Bezug genommen.

Im Allgemeinen kann die Veresterung der entsprechenden Dicarbonsäure, z.B. 4-Oxoheptandisäure, in Anwesenheit der oben beschriebenen Alkoholkomponenten R₁-OH und/oder R₂-OH mittels einer organischen Säure oder Mineralsäure, insbesondere konzentrierter Schwefelsäure, durchgeführt werden. Es kann dabei vorteilhaft sein, dass die Alkoholkomponente mindestens in der doppelten stöchiometrischen Menge, bezogen auf die Dicarbonsäure, eingesetzt wird.

Die Veresterung kann bei Umgebungsdruck oder vermindertem oder erhöhtem Druck erfolgen. Es kann bevorzugt sein, dass die Veresterung bei Umgebungsdruck oder vermindertem Druck durchgeführt wird.

Die Veresterung kann in Abwesenheit eines zugesetzten Lösungsmittels oder in Gegenwart eines Lösungsmittels durchgeführt werden.

Falls die Veresterung in Gegenwart eines Lösungsmittels durchgeführt wird, handelt es sich dabei vorzugsweise um ein unter den Reaktionsbedingungen inertes Lösungsmittel. Unter einem inerten Lösungsmittel wird in der Regel ein Lösungsmittel verstanden, welches unter den gegebenen Reaktionsbedingungen keine Reaktionen mit den an der Reaktion beteiligten Edukten, Reagenzien, Lösungsmitteln oder den entstehenden Produkten eingeht. Vorzugsweise kann das inerte Lösungsmittel ein Azeotrop mit Wasser bilden. Dazu gehören beispielsweise aliphatische Kohlenwasserstoffe, halogenierte aliphatische Kohlenwasserstoffe, aromatische und substituierte aromatische Kohlenwasserstoffe oder Ether. Es kann bevorzugt sein, dass das Lösungsmittel unter Pentan, Hexan, Heptan, Ligroin, Petrolether, Cyclohexan, Dichlormethan, Trichlormethan, Tetrachlormethan, Benzol, Toluol, Xylol, Chlorbenzol, Dichlorbenzolen, Dibutylethern, THF, Dioxan und Mischungen davon ausgewählt wird.

Die Veresterung wird üblicherweise in einem Temperaturbereich von 50 bis 250 °C durchgeführt.

Ist der Veresterungskatalysator ausgewählt unter organischen Säuren oder Mineralsäuren, wird die Veresterung üblicherweise in einem Temperaturbereich von 50 bis 160 °C durchgeführt.

Ist der Veresterungskatalysator ausgewählt unter amphoteren Katalysatoren, wird die Veresterung üblicherweise in einem Temperaturbereich von 100 bis 250 °C durchgeführt.

Die Veresterung kann in Abwesenheit oder in Gegenwart eines Inertgases erfolgen. Unter einem Inertgas wird in der Regel ein Gas verstanden, welches unter den gegebenen Reaktionsbedingungen keine Reaktionen mit den an der Reaktion beteiligten Edukten, Reagenzien, Lösungsmitteln oder den entstehenden Produkten eingeht. Es kann bevorzugt sein, dass die Veresterung ohne die Zugabe eines Inertgases erfolgt.

Beispielsweise werden der Alkohol und die Säure in einem Molverhältnis von 2:1 in einem Rührkolben zusammen mit dem Veresterungskatalysator Aluminium-tri-methyl-sulfonat in einem Molverhältnis von 400:1, bezogen auf die Säure ohne Inertgas zusammengegeben. Das Reaktionsgemisch wird zum Sieden erhitzt, vorzugsweise von 100 bis 140 °C. Das bei der Reaktion entstehende Wasser wird zusammen mit dem Alkohol azeotrop abdestilliert und anschließend abgetrennt. Der Alkohol wird wieder in das Reaktionsgemisch zurückgeführt.

Die zur Herstellung der Verbindungen der allgemeinen Formel (I) eingesetzte Dicarbonsäure und aliphatischen Alkohole können entweder kommerziell erworben oder nach literaturbekannten Synthesewegen hergestellt werden.

### Umesterung

Die Herstellung der Verbindungen der allgemeinen Formel (I) kann auch durch Umesterung erfolgen. Umesterungsverfahren und spezifische Verfahrensmaßnahmen sind entweder dem Fachmann bekannt oder erschließen sich ihm durch sein allgemeines Fachwissen. Als Edukte dienen im Allgemeinen Verbindungen der allgemeinen Formel (I), in denen R₁ und R₂ unabhängig voneinander für C₁ bis C₂-Alkyl stehen. Dazu zählt beispielsweise die Umsetzung entsprechender Carbonsäuredialkylester, beispielsweise 4-Oxoheptandisäuredimethylester oder 4-Oxoheptandisäurediethylester oder 4-Oxoheptandisäureethylmethylester oder Mischungen davon, mit mindestens einer Alkoholkomponente ausgewählt aus den Alkoholen R₁-OH und R₂-OH, wobei R₁ und R₂ für C₁₀-C₁₃-Alkyl stehen, wobei zumindest ein Teil der Reste R₁ und/oder R₂ verzweigt ist, in Anwesenheit eines geeigneten Umesterungskatalysators.

Als Umesterungskatalysatoren kommen beispielsweise die üblichen, gewöhnlich für Umesterungsreaktionen verwendeten Katalysatoren in Betracht, die meist auch bei Veresterungsreaktionen eingesetzt werden. Hierzu zählen z.B. Mineralsäuren, wie Schwefelsäure oder Phosphorsäure; organische Sulfonsäuren, wie Methansulfonsäure oder p-Toluolsulfonsäure; oder spezielle Metallkatalysatoren aus der Gruppe der Zinn-(IV)-Katalysatoren, beispielsweise Dialkylzinndicarboxylate wie Dibutylzinndiacetat, Trialkylzinnalkoxide, Monoalkylzinnverbindungen wie Monobutylzinndioxid, Zinnsalze wie Zinnacetat oder Zinnoxide; aus der Gruppe der Titankatalysatoren, monomere oder polymere Titanate oder Titanchelate wie Tetraethylorthotitanat, Tetrapropylorthotitanat, Tetrabutylorthotitanat, Triethanolamintitanat; aus der Gruppe der Zirkonkatalysatoren, Zirkonate oder Zirkonchelate wie Tetrapropylzirkonat, Tetrabutylzirkonat, Triethanolaminzirkonat; sowie Lithiumkatalysatoren wie Lithiumsalze, Lithiumalkoxide; oder Aluminium(III)-, Chrom(III)-, Eisen(III)-, Kobalt(II)-, Nickel(II) und Zink(II)-acetylacetonat.

Die Menge an eingesetztem Umesterungskatalysator kann im Allgemeinen bei 0,001 bis 10 Gew.-%, bevorzugt 0,05 bis 5 Gew.-% liegen. Das Reaktionsgemisch wird in der Regel bis zum Siedepunkt des Reaktionsgemisches erhitzt, so dass die Reaktionstemperatur in Abhängigkeit von den Reaktanden in einem Temperaturbereich von 20 bis 200 °C liegt.

Die Umesterung kann bei Umgebungsdruck oder vermindertem oder erhöhtem Druck erfolgen. Es kann bevorzugt sein, dass die Umesterung bei einem Druck von 0,001 bis 200 bar, stärker bevorzugt 0,01 bis 5 bar, durchgeführt wird.

Der bei der Umesterung abgespaltene, niedriger siedende Alkohol kann zwecks Verschiebung des Gleichgewichts der Umesterungsreaktion kontinuierlich abdestilliert werden. Die hierzu benötigte Destillationskolonne steht in der Regel in direkter Verbindung mit dem Umesterungsreaktor. Beispielsweise kann die Destillationskolonne direkt am Umesterungsreaktor installiert sein. Im Falle der Verwendung mehrerer, in Serie geschalteter Umesterungsreaktoren kann jeder dieser Reaktoren mit einer Destillationskolonne ausgerüstet sein oder es kann, vorzugsweise aus den letzten Kesseln der Umesterungsreaktorkaskade, das abgedampfte Alkoholgemisch über eine oder mehrere Sammelleitungen einer Destillationskolonne zugeführt werden. Der bei dieser Destillation zurückgewonnene höhersiedende Alkohol wird vorzugsweise wieder in die Umesterung zurückgeführt.

Im Falle der Verwendung eines amphoteren Katalysators gelingt dessen Abtrennung im Allgemeinen durch Hydrolyse und anschließende Abtrennung des gebildeten Metalloxids, z.B. durch Filtration. Es kann bevorzugt sein, dass nach erfolgter Reaktion der Katalysator mittels Waschen mit Wasser hydrolysiert und das ausgefallene Metalloxid abfiltriert wird. Das Filtrat kann einer weiteren Aufarbeitung zur Isolierung und/oder Reinigung des Produkts unterzogen werden. Es kann bevorzugt sein, dass das Produkt destillativ abgetrennt wird.

Die Umesterung der Di-(C₁-C₂)-Alkylester entsprechender Dicarbonsäuren, beispielsweise 4-Oxoheptandisäuredimethylester, mit mindestens einer Alkoholkomponente, ausgewählt aus den Alkoholen R₁-OH und R₂-OH kann bevorzugt in Gegenwart mindestens eines Titan (IV)-Alkoholats erfolgen. Bevorzugte Titan (IV)-Alkoholate sind Tetrapropoxytitan, Tetrabutoxytitan oder Gemische davon. Es kann bevorzugt sein, dass die Alkoholkomponente mindestens in der doppelten stöchiometrischen Menge, bezogen auf die eingesetzten Di-(C₁-C₂-Alkyl)-Ester eingesetzt wird.

Die Umesterung kann in Abwesenheit oder in Gegenwart eines zugesetzten Lösungsmittels durchgeführt werden. Es kann bevorzugt sein, dass die Umesterung in Gegenwart eines inerten Lösungsmittels durchgeführt wird. Geeignete Lösungsmittel sind die zuvor für die Veresterung genannten. Dazu zählen speziell Toluol und THF.

Die Temperatur bei der Umesterung liegt in der Regel in einem Bereich von 20 bis 200 °C.

Die Umesterung kann in Abwesenheit oder in Gegenwart eines Inertgases erfolgen. Unter einem Inertgas wird in der Regel ein Gas verstanden, welches unter den gegebenen Reaktionsbedingungen keine Reaktionen mit den an der Reaktion beteiligten Edukten, Reagenzien, Lösungsmitteln oder den entstehenden Produkten eingeht. Es kann bevorzugt sein, dass die Umesterung ohne Hinzufügen eines Inertgases durchgeführt wird.

Bevorzugte C₁₀ bis C₁₃-Alkanole, die zur Herstellung der in der erfindungsgemäßen Weichmacher-Zusammensetzung enthaltenen Verbindungen (I) eingesetzt werden, können verzweigt sein oder aus Gemischen aus geradkettigen und verzweigten C₁₀ bis C₁₃-Alkanolen bestehen. Dazu zählen Isodecanol, 2-Propylheptanol, 3,7-Dimethyl-1-octanol, Isoundecanol, Isododecanol oder Isotridecanol. Es kann bevorzugt sein, dass C₁₀ bis C₁₂-Alkanole wie 2-Propylheptanol oder 3,7-Dimethyl-1-octanol eingesetzt werden und stärker bevorzugt, dass 2-Propylheptanol eingesetzt wird.

### Decanol

Die Decanole, die zur Herstellung der in der Weichmacher-Zusammensetzung enthaltenen Verbindungen der allgemeinen Formel (I) eingesetzt werden, können verzweigt sein oder aus Gemischen geradkettiger und verzweigter Decanole zusammengesetzt sein. Es kann bevorzugt sein, dass Gemische aus verzweigten Decanolen, auch als Isodecanol bezeichnet, als Alkoholkomponente verwendet werden.

Bei Isodecanol, welches zur Synthese der in der Weichmacher-Zusammensetzung enthaltenen Diisodecylester der allgemeinen Formel (I) eingesetzt wird, handelt es sich in der Regel nicht um eine einheitliche chemische Verbindung, sondern um ein komplexes Gemisch unterschiedlich verzweigter isomerer Decanole.

Diese werden im Allgemeinen durch die Nickel- oder Brønsted-Säure-katalysierte Trimerisierung von Propylen, beispielsweise nach dem vorstehend erläuterten PolyGas^{®}- oder dem EMOGAS^{®}-Verfahren, nachfolgende Hydroformylierung des dabei erhaltenen Isononen-Isomerengemisches mittels homogener Rhodium- oder Kobaltcarbonyl-Katalysatoren, vorzugsweise mittels Kobaltcarbonyl-Katalysatoren und Hydrierung des entstandenen Isodecanal-Isomerengemisches, z.B. mittels der vorstehend in Zusammenhang mit der Herstellung von C₇-C₉-Alkoholen genannten Katalysatoren und Verfahren (Ullmann's Encyclopedia of Industrial Chemistry; 5. Auflage, Bd. A1, S. 293, VCH Verlagsgesellschaft GmbH, Weinheim 1985), hergestellt. Das so produzierte Isodecanol ist im Allgemeinen stark verzweigt.

Bei 2-Propylheptanol, welches zur Synthese der in der Weichmacher-Zusammensetzung enthaltenen Di-(2-propylheptyl)ester der allgemeinen Formel (I) eingesetzt wird, kann es sich um reines 2-Propylheptanol handeln oder um Propylheptanol-Isomerengemische, wie sie im Allgemeinen bei der industriellen Herstellung von 2-Propylheptanol gebildet werden und gemeinhin ebenfalls als 2-Propylheptanol bezeichnet werden.

Reines 2-Propylheptanol kann beispielsweise durch Aldolkondensation von n-Valeraldehyd und nachfolgende Hydrierung des dabei gebildeten 2-Propylheptenals, beispielsweise gemäß US-A 2921089, erhalten werden. Im Allgemeinen enthält kommerziell erhältliches 2-Propylheptanol neben der Hauptkomponente 2-Propylheptanol herstellungsbedingt eines oder mehrere der 2-Propylheptanol-Isomeren 2-Propyl-4-methylhexanol, 2-Propyl-5-methylhexanol, 2-Isopropyl-heptanol, 2-Isopropyl-4-methylhexanol, 2-Isopropyl-5-methylhexanol und/oder 2-Propyl-4,4-dimethylpentanol. Die Anwesenheit anderer Isomere des 2-Propylheptanols, beispielsweise 2-Ethyl-2,4-dimethylhexanol, 2-Ethyl-2-methyl-heptanol und/oder 2-Ethyl-2,5-dimethylhexanol im 2-Propylheptanol, ist möglich, aufgrund der geringen Bildungsraten der aldehydischen Vorläufer dieser Isomere im Zuge der Aldolkondensation sind diese, wenn überhaupt, nur in Spurenmengen im 2-Propylheptanol enthalten und spielen für die Weichmachereigenschaften des aus solchen 2-Propyheptanol-Isomerengemischen hergestellten Verbindungen praktisch keine Rolle.

Als Ausgangsmaterial zur Herstellung von 2-Propylheptanol können verschiedenerlei Kohlenwasserstoffquellen benutzt werden, beispielsweise 1-Buten, 2-Buten, Raffinat I (ein aus dem C₄-Schnitt eines Crackers nach Abtrennung von Allenen, Acetylenen und Dienen erhaltenes Alkan/Alken-Gemisch, das neben 1- und 2-Buten noch erhebliche Mengen an Isobuten enthält) oder Raffinat II, das aus Raffinat I durch Abtrennung von Isobuten erhalten wird und als Olefinkomponenten außer 1- und 2-Buten nur noch geringe Anteile an Isobuten enthält. Selbstverständlich können auch Gemische aus Raffinat I und Raffinat II als Rohstoff zur 2-Propylheptanol-Herstellung verwendet werden. Diese Olefine oder Olefingemische können nach an sich herkömmlichen Methoden mit Kobalt- oder Rhodium-Katalysatoren hydroformyliert werden, wobei aus 1-Buten ein Gemisch aus n- und iso-Valeraldehyd (die Bezeichnung iso-Valeraldehyd bezeichnet die Verbindung 2-Methylbutanal) gebildet wird, dessen n/iso-Verhältnis je nach verwendetem Katalysator und Hydroformylierungsbedingungen in relativ weiten Grenzen variieren kann. Beispielsweise wird bei Verwendung eines mit Triphenylphosphin modifizierten homogenen Rhodium-Katalysators (Rh/TPP) aus 1-Buten n- und iso-Valeraldehyd in einem n/iso-Verhältnis von im Allgemeinen 10 : 1 bis 20 : 1 gebildet, wohingegen bei Verwendung von mit Phosphit-Liganden, beispielsweise gemäß US-A 5288918 oder WO 05028407, oder von mit Phosphoamidit-Liganden, beispielsweise gemäß WO 0283695, modifizierten Rhodium-Hydroformylierungskatalysatoren fast ausschließlich n-Valeraldehyd gebildet wird. Während das Rh/TPP-Katalysatorsystem 2-Buten bei der Hydroformylierung nur sehr langsam umsetzt, so dass der größte Teil des 2-Butens aus dem Hydroformylierungsgemisch wieder zurückgewonnen werden kann, gelingt die Hydroformylierung des 2-Butens mit den erwähnten Phosphit-Ligand- oder Phosphoramidit-Ligand-modifizierten Rhodium-Katalysatoren, wobei vorwiegend n-Valeraldehyd gebildet wird. Hingegen wird im olefinischen Rohstoff enthaltenes Isobuten, wenn auch mit unterschiedlicher Geschwindigkeit, von praktisch allen Katalysatorsystemen zu 3-Methylbutanal und je nach Katalysator in geringerem Umfang zu Pivalaldehyd hydroformyliert.

Die je nach verwendeten Ausgangsmaterialien und Katalysatoren erhaltenen C₅-Aldehyde, d.h. n-Valeraldehyd gegebenenfalls im Gemisch mit iso-Valeraldehyd, 3-Methylbutanal und/oder Pivalaldehyd, können vor der Aldolkondensation gewünschtenfalls vollständig oder teilweise destillativ in die Einzelkomponenten aufgetrennt werden, so dass auch hier eine Möglichkeit besteht, die Isomerenzusammensetzung der C₁₀-Alkoholkomponente der offenbarungsgemäßen Estergemische zu beeinflussen und zu steuern. Desgleichen ist es möglich, das C₅-Aldehydgemisch, wie es bei der Hydroformylierung gebildet wird, ohne die vorherige Abtrennung einzelner Isomere der Aldolkondensation zuzuführen. Bei der Aldolkondensation, die mittels eines basischen Katalysators, wie einer wässrigen Lösung von Natrium- oder Kaliumhydroxid, beispielsweise nach den in EP-A 366089, US-A 4426524 oder US-A 5434313 beschriebenen Verfahren durchgeführt werden kann, entsteht bei Einsatz von n-Valeraldehyd als einziges Kondensationsprodukt 2-Propylheptenal, wohingegen bei Einsatz eines Gemisches isomerer C₅-Aldehyde ein Isomerengemisch aus den Produkten der Homoaldolkondensation gleicher Aldehydmoleküle und der gekreuzten Aldolkondensation unterschiedlicher Valeraldehyd-Isomere geformt wird. Selbstverständlich kann die Aldolkondensation durch die gezielte Umsetzung einzelner Isomere so gesteuert werden, dass überwiegend oder vollständig ein einzelnes Aldolkondensationsisomer gebildet wird. Die betreffenden Aldolkondensationsprodukte können anschließend, üblicherweise nach vorausgegangener, meist destillativer Abtrennung aus der Reaktionsmischung und gewünschtenfalls destillativer Reinigung, mit herkömmlichen Hydrierkatalysatoren, beispielsweise den vorstehend zur Hydrierung von Aldehyden genannten, zu den entsprechenden Alkoholen oder Alkoholgemischen hydriert werden.

Wie bereits erwähnt, können die in der Weichmacher-Zusammensetzung enthaltenen Verbindungen der allgemeinen Formel (I) mit reinem 2-Propylheptanol verestert sein. Im Allgemeinen werden zur Herstellung dieser Ester jedoch Gemische des 2-Propylheptanols mit den genannten Propylheptanol-Isomeren eingesetzt, in denen der Gehalt an 2-Propylheptanol mindestens 50 Gew.-% Beträgt. Es kann bevorzugt sein, dass der Gehalt an 2-Propylheptanol 60 bis 98 Gew.-% und stärker bevorzugt 80 bis 95 Gew.-% und besonders bevorzugt 85 bis 95 Gew.-% beträgt.

Geeignete Mischungen von 2-Propylheptanol mit den Propylheptanol-Isomeren umfassen beispielsweise solche aus 60 bis 98 Gew.-% 2-Propylheptanol, 1 bis 15 Gew.-% 2-Propyl-4-methyl-hexanol und 0,01 bis 20 Gew.-% 2-Propyl-5-methyl-hexanol und 0,01 bis 24 Gew.-% 2-Isopropylheptanol, wobei die Summe der Anteile der einzelnen Bestandteile 100 Gew.-% nicht überschreitet. Es kann bevorzugt sein, dass sich die Anteile der einzelnen Bestandteile zu 100 Gew.-% addieren.

Weitere geeignete Mischungen aus 2-Propylheptanol mit den Propylheptanol-Isomeren umfassen beispielsweise solche aus 75 bis 95 Gew.-% 2-Propylheptanol, 2 bis 15 Gew.-% 2-Propyl-4-methyl-hexanol, 1 bis 20 Gew.-% 2-Propyl-5-methyl-hexanol, 0,1 bis 4 Gew.-% 2-Isopropylheptanol, 0,1 bis 2 Gew.-% 2-Isopropyl-4-methylhexanol und 0,1 bis 2 Gew.-% 2-Isopropyl-5-methyl-hexanol, wobei die Summe der Anteile der einzelnen Bestandteile 100 Gew.-% nicht überschreitet. Es kann bevorzugt sein, dass sich die Anteile der einzelnen Bestandteile zu 100 Gew.-% addieren.

Es kann bevorzugt sein, dass Mischungen von 2-Propylheptanol mit den Propylheptanol-Isomeren solche mit 85 bis 95 Gew.-% 2-Propylheptanol, 5 bis 12 Gew.-% 2-Propyl-4-methyl-hexanol und 0,1 bis 2 Gew.-% 2-Propyl-5-methylhexanol und 0,01 bis 1 Gew.-% 2-Isopropylheptanol umfassen, wobei die Summe der Anteile der einzelnen Bestandteile 100 Gew.-% nicht überschreitet. Es kann bevorzugt sein, dass sich die Anteile der einzelnen Bestandteile zu 100 Gew.-% addieren.

Bei Verwendung der genannten 2-Propylheptanol-Isomerengemische anstelle von reinem 2-Propylheptanol zur Herstellung der Verbindungen der allgemeinen Formel (I) entspricht die Isomerenzusammensetzung der Alkylestergruppen bzw. Alkylethergruppen praktisch der Zusammensetzung der zur Veresterung verwendeten Propylheptanol-Isomerengemische.

### Undecanol

Die Undecanole, die zur Herstellung der in der Weichmacher-Zusammensetzung enthaltenen Verbindungen der allgemeinen Formel (I) eingesetzt werden, können verzweigt sein oder aus Gemischen geradkettiger und verzweigter Undecanole zusammengesetzt sein. Es kann bevorzugt sein, dass Gemische aus verzweigten Undecanolen, auch als Isoundecanol bezeichnet, als Alkoholkomponente verwendet werden.

Im Wesentlichen geradkettiges Undecanol kann beispielsweise durch die Rhodium- oder vorzugsweise Kobalt-katalysierte Hydroformylierung von 1-Decen und nachfolgende Hydrierung des dabei erhaltenen n-Undecanals erhalten werden. Das Ausgangsolefin 1-Decen wird beispielsweise über das zuvor bei der Herstellung von 1-Octen erwähnte SHOP-Verfahren hergestellt.

Zur Herstellung verzweigten Isoundecanols kann das im SHOP-Verfahren erhaltene 1-Decen einer Skelettisomerisierung, z.B. mittels azider zeolithischer Molekularsiebe, wie in WO 9823566 beschrieben, unterzogen werden, wobei sich Gemische aus isomeren Decenen bilden, deren Rhodium- oder vorzugsweise Kobalt-katalysierte Hydroformylierung und nachfolgende Hydrierung der erhaltenen Isoundecanal-Gemische zudem zur Herstellung der offenbarungsgemäßen Verbindungen der allgemeinen Formel (I) verwendeten Isoundecanols führt. Die Hydroformylierung von 1-Decen oder Isodecen-Gemischen mittels Rhodium- oder Kobalt-Katalyse kann wie zuvor in Zusammenhang mit der Synthese von C₇ bis C₁₀-Alkoholen beschrieben erfolgen. Entsprechendes gilt für die Hydrierung von n-Undecanal oder Isoundecanal-Gemischen zu n-Undecanol bzw. Isoundecanol.

Nach destillativer Reinigung des Austrags der Hydrierung können die so erhaltenen C₇ bis C₁₁-Alkylalkohole bzw. deren Gemische, wie vorstehend beschrieben, zur Herstellung der offenbarungsgemäßen Diesterverbindungen der allgemeinen Formel (I) verwendet werden.

### Dodecanol

Die Dodecanole, die zur Herstellung der in der Weichmacher-Zusammensetzung enthaltenen Verbindungen der allgemeinen Formel (I) eingesetzt werden, können verzweigt sein oder aus Gemischen geradkettiger und verzweigter Dodecanole zusammengesetzt sein.

Im Wesentlichen geradkettiges Dodecanol kann beispielsweise über das Alfol^{®}- oder Epal^{®}-Verfahren gewonnen werden. Diese Verfahren beinhalten die Oxidation und Hydrolyse geradkettiger Trialkylaluminium-Verbindungen, welche ausgehend von Triethylaluminium schrittweise über mehrere Ethylierungsreaktionen unter Verwendung von Ziegler-Natta-Katalysatoren aufgebaut werden. Aus den daraus resultierenden Gemischen weitgehend geradkettiger Alkylalkohole unterschiedlicher Kettenlänge kann nach dem destillativen Austrag der C₁₂-Alkylalkohol-Fraktion das gewünschte n-Dodecanol erhalten werden.

Alternativ kann n-Dodecanol auch durch Hydrogenierung natürlicher Fettsäuremethylester, beispielsweise aus Kokosnussöl, hergestellt werden.

Verzweigtes Isododecanol kann analog zu den bekannten Verfahren zur Codimerisierung und/oder Oligomerisierung von Olefinen, wie beispielsweise in der WO 0063151 beschrieben, mit nachfolgender Hydroformylierung und Hydrierung der Isoundecen-Gemische, wie beispielsweise in der DE-A 4339713 beschrieben, erhalten werden. Nach destillativer Reinigung des Austrags der Hydrierung können die so erhaltenen Isododecanole bzw. deren Gemische, wie vorstehend beschrieben, zur Herstellung der offenbarungsgemäßen Diesterverbindungen der allgemeinen Formel (I) verwendet werden.

### Tridecanol

Die Tridecanole, die zur Herstellung der in der Weichmacher-Zusammensetzung enthaltenen Verbindungen der allgemeinen Formel (I) eingesetzt werden, können verzweigt sein oder aus Gemischen geradkettiger und verzweigter Tridecanole zusammengesetzt sein.

Die Synthese von Isotridecanolen ist in EP 1 230 200 beschrieben, auf welche vollumfänglich Bezug genommen wird. Das C₁₃-Alkoholgemisch wird dadurch erhalten, dass man a) einen Butene enthaltenden C₄-Kohlenwasserstoffstrom, der weniger als 5 Gew.-%, bezogen auf die Butenfraktion iso-Buten enthält, bei erhöhter Temperatur mit einem Nickel enthaltenden heterogenen Katalysator in Kontakt bringt, b) aus dem Reaktionsgemisch eine C₁₂-Olefinfraktion isoliert, c) die C₁₂-Olefinfraktion durch Umsetzung mit Kohlenmonoxid und Wasserstoff in Gegenwart eines Kobalt-Katalysators hydroformyliert und d) hydriert.

### Beispiele

Die Erfindung wird anhand der im Folgenden beschriebenen Figuren und der Beispiele näher erläutert. Dabei sollen die Figuren und Beispiele nicht als einschränkend für die Erfindung verstanden werden.

Die erfindungsgemäßen Weichmacher haben eine sehr niedrige Folienflüchtigkeit, welche den Einsatz dieser Verbindungen in anspruchsvollen Anwendungen, wie z.B. im Automobilinnenbereich ermöglichen, und das bei gleicher, sehr niedriger Kältebruchtemperatur und ansonsten ausgewogenem Eigenschaftsprofil.

Für die Gaschromatographie wurde ein Gaschromatograph von Fa. Agilent 6890series und als Säule Optima 5 Amin (Länge = 30 m, Innendurchmesser = 0,25 mm, Außendurchmesser = 0,40 mm, Filmdicke = 0,5 µm) der Fa. Macherey&Nagel (Best.Nr. 726354.30) verwendet. Als Injektor diente ein Split/Splitless mit Topaz Split Precision Liner Whool der Fa. Restek (# 23305). Die Injektionsbedingungen waren: Injektortemperatur = 280 °C, Injektionsvolumen = 1 µL, Split 1:50, Splitflow 150 mL/min, Septum Purge 3,0 mL/min (gemessen bei Ofentemperatur 80 °C). Als Trägergas diente Stickstoff 28PSI = 3,0 mL/min (gemessen bei Ofentemperatur 80 °C). Das Temperaturprogramm war: Start: 60 °C, Verweildauer 1: 5 min, Temperaturrampe 1: 8 °C/min, Endtemperatur 1: 240 °C, Verweildauer 2: 0 min, Temperaturrampe 2: 30 °C/min, Endtemperatur 2: 300 °C, Verweildauer 3: 10 min, Gesamtlaufzeit: 59,5 min. Die Detektion erfolgte mittels FID mit 300 mL/min Luft, 30 mL/min Wasserstoff und 30 mL/min Make-Up-Gas (Stickstoff) bei 320 °C.

In den Beispielen werden die Einsatzstoffe wie in Tabelle 2 gezeigt verwendet.

**Tabelle 2.**

| Einsatzstoffe | Beispielsweise kommerziell erhältlich | |
|---|---|---|
| | als | von |
| Homopolymeres Emulsions-PVC | Vinoflex S7114 | Inovyn Limited |
| Homopolymeres Suspensions-PVC | Inovyn 271 PC | Inovyn Limited |
| Ba-Zn Stabilisator | Baerostab^{®} UBZ 760 XLP RF | Baerlocher Italia SPA. |
| Diethyl-4-oxopimelat | | SigmaAldrich |
| 2-Propylheptanol | | BASF SE |
| 3,7-Dimethyl-1-Octanol | | SigmaAldrich |
| Isotridecanol | Isotridecanol N | BASF SE |
| 2-Ethylhexanol | | SigmaAldrich |
| Titan-Veresterungskatalysator | Tyzor TPT-20B | Dorf Ketal bv, Eindhoven, Niederlande |

### Synthese der Verbindung I.1 (4-Oxoheptandisäure-di-(2-propylheptylester))

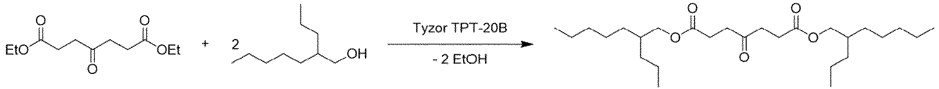

Ein 1.6 L Reaktorkessel wurde mit Diethyl-4-oxopimelat (500 g, 2.17 mol, 1.0 Äq.), 2-Propylheptanol (962 g, 6.08 mol, 2.8 Äq.) und Tyzor TPT-20B (0.73 g, 0.05 Gew.-t%) befüllt. Unter einem Stickstoff-Strom wurde die Reaktionsmischung auf 144 °C geheizt, um zum einen die Reaktionsmischung am Sieden zu halten und zum anderen das entstandene Ethanol kontinuierlich abzudestillieren. Nach 3.25 Stunden Reaktionszeit war kein Ethanol mehr entstanden. Anschließend wurde das überschüssige 2-Propylheptanol abdestilliert (max. 220 °C, Stickstoff-Strom: 10 L/h, 13 mbar). Die Reaktionstemperatur wurde auf 80 °C gebracht und die Reaktionsmischung mit einer wässrigen 2.0 Gew.-% NaOH-Lösung (5.0 g) gequencht. Nach 20-minütigem Rühren bei 80 °C wurde der Reaktionsmischung Wasser (47 mL) hinzugefügt, um das ausgefallene Titandioxid zu agglomerieren. Als nächstes wurde das Wasser abdestilliert (max. 125 °C, 8 mbar), die Reaktionsmischung auf Raumtemperatur abgekühlt und über eine Druckfilternutsche filtriert (2 L Pall Filterhalter, Filterplatte AKS 7 mit Aktivkohle). Danach wurde eine Wasserdampfdestillation (max. 200 °C, Dauer: 1 h) durchgeführt und als letztes wurde ein Stickstoff-Strom durch das Produkt geleitet (200 °C, 20 min). Um das Produkt hochrein zu erhalten, wurden 400 g des Rohprodukts weitere 12.5 h am Wasserdampfstrip bei 200 °C unter kräftiger Stickstoffdurchleitung (ca. 25 L/h) aufgereinigt. Anschließend wurde ein Stickstoff-Strom durch das Produkt geleitet (200 °C, 20 min). Es wurden 330 g des Produkts erhalten (726 mmol, 39% Ausbeute). Analytik: GC-Flächen%: 98.5%.

### Synthese der Verbindung I.2 (4-Oxoheptandisäure-di-(3,7-dimethyloctylester))

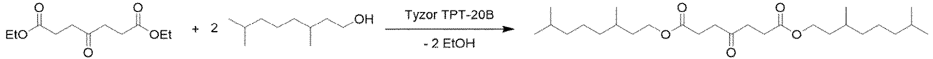

Ein 1.6 L Reaktorkessel wurde mit Diethyl-4-oxopimelat (320 g, 1.39 mol, 1.0 Äq.), 3,7-Dimethyl-1-Octanol (660 g, 4.17 mol, 3.0 Äq.) und Tyzor TPT-20B (0.49 g, 0.05 Gew.-%) befüllt. Unter einem milden Stickstoff-Strom wurde die Reaktionsmischung auf 200 °C (Band Limitierung: 25 K) geheizt, um zum einem die Reaktionsmischung am Sieden zu halten und zum anderen das entstandene Ethanol kontinuierlich abzudestillieren. Nach 3 Stunden Reaktionszeit war kein Ethanol mehr entstanden. Anschließend wurde das überschüssige 3,7-Dimethyl-1-Octanol abdestilliert (200 °C, 7 mbar). Die Reaktionstemperatur wurde auf 80 °C gebracht und die Reaktionsmischung mit einer wässrigen 2.0 Gew.-% NaOH-Lösung (3.5 g) gequencht. Nach 30-minütigem Rühren bei 80 °C wurde der Reaktionsmischung Wasser (33 mL) hinzugefügt, um das ausgefallene Titandioxid zu agglomerieren. Als nächstes wurde das Wasser abdestilliert (86 °C, 18 mbar), die Reaktionsmischung auf Raumtemperatur abgekühlt und über eine Druckfilternutsche filtriert (2 L Pall Filterhalter, Filterplatte KS 80). Danach wurde eine Wasserdampfdestillation (160 - 195 °C, Dauer: 1.25 h) durchgeführt und als letztes wurde ein Stickstoff-Strom durch das Produkt geleitet (200 °C, 20 min), um das Produkt hochrein zu erhalten. Das Produkt wurde als gelbe Flüssigkeit erhalten (546 g, 1.20 mol, 86% Ausbeute). Analytik: GC-Flächen%: 95.2%.

### Synthese der Verbindung I.6 (4-Oxoheptandisäure-di-isotridecylester)

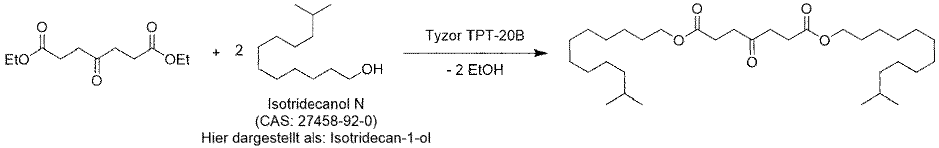

Ein 1.6 L Reaktorkessel wurde mit Diethyl-4-oxopimelat (320 g, 1.39 mol, 1.0 Äq.), Isotridecanol N (835 g, 4.17 mol, 3.0 Äq.) und Tyzor TPT-20B (0.58 g, 0.05 Gew.-%) befüllt. Unter einem milden Stickstoff-Strom wurde die Reaktionsmischung auf 200 °C (Band Limitierung: 25 K) geheizt, um zum einem die Reaktionsmischung am Sieden zu halten und zum anderen das entstandene Ethanol kontinuierlich abzudestillieren. Nach 3 Stunden Reaktionszeit war kein Ethanol mehr entstanden. Anschließend wurde das überschüssige Isotridecanol N abdestilliert (209 °C, 7 mbar). Die Reaktionstemperatur wurde auf 80 °C gebracht und die Reaktionsmischung mit einer wässrigen 2.0 Gew.-% NaOH-Lösung (4.0 g) gequencht. Nach 30-minütigem Rühren bei 80 °C wurde der Reaktionsmischung Wasser (37 mL) hinzugefügt, um das ausgefallene Titandioxid zu agglomerieren. Als nächstes wurde das Wasser abdestilliert (86 °C, 18 mbar), die Reaktionsmischung auf Raumtemperatur abgekühlt und über einer Druckfilternutsche filtriert (2 L Pall Filterhalter, Filterplatte AKS 70). Danach wurde eine Wasserdampfdestillation (154 - 179 °C, Dauer: 2.0 h) durchgeführt und als letztes wurde ein Stickstoff-Strom durch das Produkt geleitet (200 °C, 20 min), um das Produkt hochrein zu erhalten. Das Produkt wurde als gelbe viskose Flüssigkeit erhalten (615 g, 1.14 mol, 82% Ausbeute). Analytik: GC-Flächen%: 98.3%.

### Synthese der Verbindung 4-Oxoheptandisäure-di-(2-ethylhexylester) (Vergleichsbeispiel)

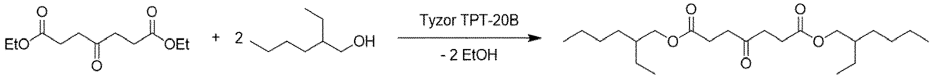

Ein 1.6 L Reaktorkessel wurde mit Diethyl-4-oxopimelat (500 g, 2.17 mol, 1.0 Äq.), 2-Ethylhexanol (848 g, 6.51 mol, 3.0 Äq.) und Tyzor TPT-20B (0.73 g, 0.05 Gew.-%) befüllt. Unter einem Stickstoff-Strom wurde die Reaktionsmischung auf 145 °C geheizt, um zum einem die Reaktionsmischung am Sieden zu halten und zum anderen das entstandene Ethanol kontinuierlich abzudestillieren. Nach 7 Stunden Reaktionszeit war kein Ethanol mehr entstanden. Anschließend wurde das überschüssige 2-Propylheptanol abdestilliert (max. 195 °C, Stickstoff-Strom: 10 L/h, 9 mbar). Die Reaktionstemperatur wurde auf 80 °C gebracht und die Reaktionsmischung mit einer wässrigen 2.0 Gew.-% NaOH-Lösung (5.0 g) gequencht.

Nach 20-minütigem Rühren bei 80 °C wurde der Reaktionsmischung Wasser (47 mL) hinzugefügt, um das ausgefallene Titandioxid zu agglomerieren. Als nächstes wurde das Wasser abdestilliert (max. 125 °C, 8 mbar), die Reaktionsmischung auf Raumtemperatur abgekühlt und über eine Druckfilternutsche filtriert (2 L Pall Filterhalter, Filterplatte AKS 7 mit Aktivkohle). Danach wurde eine Wasserdampfdestillation (max. 200 °C, Dauer: 6 h) durchgeführt und als letztes wurde ein Stickstoff-Strom durch das Produkt geleitet (200 °C, 10 min). Um das Produkt hochrein zu erhalten, wurde das Rohprodukt weitere 7.5 h am Wasserdampfstrip bei 200 - 205 °C unter kräftiger Stickstoffdurchleitung (ca. 25 L/h) aufgereinigt. Anschließend wurde ein Stickstoff-Strom durch das Produkt geleitet (200 °C, 10 min). Es wurden 569 g des Produkts erhalten (1,43 mol, 66% Ausbeute). Analytik: GC-Flächen%: 97.9%.

Die nachfolgende Tabelle 3 gibt die Eigenschaften der wie oben beschriebenen Verbindungen wieder.

**Tabelle 3.**

| | | Verbindung I.1 | Verbindung I.2 | Verbindung I.6 | |
|---|---|---|---|---|---|
| Produkteigenschaft | Methode | (4-Oxoheptandisäure-di-(2-propylheptylester)) | (4-Oxoheptandisäure-di-(3,7-dimethyloctylester)) | (4-Oxoheptandisäure-di-isotridecylester) | 4-Oxoheptandisäure-di-(2-ethylhexylester)* |
| Dichte, 20 °C, [g/cm³] | DIN 51757 Verf. 4, 01/11 | 0,9473 | 0,9456 | 0,9365 | 0,9661 |
| Viskosität, 20 °C [mPa·s] | DIN 51562-1, 01/99 | 51 | 52 | 101 | 32 |
| Brechzahl, n^{D}₂₀ | DIN 51423-2, 02/10 | 1,4553 | 1,5160 | 1,4605 | 1,4534 |
| Säurezahl, [mg KOH/g] | DIN EN ISO 2114, 06/02 | 1,1 | 0,26 | 0,31 | 1,5 |
| Wassergehalt [Gew.-%] | DIN 51777-1, 03/83 | 0,003 | 0,013 | 0,016 | 0,07 |
| GC-Reinheit [%] | BASF-Methode | 99,8 | 95 | 98 | 98 |
| Lösetemperatur [°C] | BASF-Methode | 139 | 147 | 162 | 127 |

| | | | | | |
|---|---|---|---|---|---|
| * Vergleichsbeispiel | | | | | |

Anwendungstechnische Prüfungen:
II.a) Bestimmung der Lösetemperatur der offenbarungsgemäßen Weichmacher-Zusammensetzungen:
Zur Bestimmung der Lösetemperatur der offenbarungsgemäßen Weichmacher-Zusammensetzungen wurden etwa 10 Gramm einer Mischungen nach folgender Rezeptur (siehe Tabelle 4) hergestellt. Die Mischung wird mit einer Pipette aufgerührt und dann sofort ca. 30 Tropfen der homogenen Mischung auf das Platte-Platte-Messsystem gegeben.

**Tabelle 4.**

| | phr |
|---|---|
| PVC, K-Wert 71, gesiebte Ware mit Sieb 100 µm (PVC Typ Vinoflex^{®} S 7114) | 5 |
| offenbarungsgemäße Weichmacher-Zusammensetzung | 95 |
| Ba-Zn Stabilisator, Typ Baerostab UBZ 760 XLP RF | 2 |

Die Viskositätsmessungen wurden mit einem beheizbaren Oszillations- und Rotations-Rheometer MCR 302 von Anton Paar in einem Rotationsversuch durchgeführt.

| | |
|---|---|
| Messsystem: | Platte/Platte d=50 mm |
| Scherrate D: | 10 (1/s) |
| Spaltbreite: | 0,25 mm |
| Ausgangstemperatur: | 30 °C |
| Temperaturprofil: | 30 - 180 °C |
| Temperatursteigerung: | 5 °C/min |
| Wertaufzeichnung: | alle 3 s |

Die Messung erfolgte in zwei Rampen. Die erste Rampe für die Dauer von 120 s bei D=10 (1/s) und 30 °C dient zum Temperieren der Probe. Die zweite Rampe bei D=10 (1/s) und kontinuierlicher Temperatursteigerung von 5 °C min ist dann die eigentliche Messung. Der Abbruch erfolgte manuell, nachdem das Viskositätsmaximum überschritten war. Als Ergebnis der Messung wird die Temperatur ermittelt, bei der das Maximum der Viskosität erreicht ist. Diese Messungen werden insgesamt viermal durchgeführt und der arithmetische Mittelwert aller vier Messungen als das Endergebnis gewertet.
Il.b) Herstellung und Prüfung von Weich-PVC-Folien hergestellt unter Einsatz erfindungsgemäßer Weichmacher-Zusammensetzungen

Rezeptur: siehe nachfolgende Tabelle 5.

**Tabelle 5.**

| Additiv | phr |
|---|---|
| PVC (Homopolymeres Suspensions-PVC, Markenname Inovyn^{®} 271 PC) | 100 |
| Erfindungsgemäße Weichmacher-Zusammensetzung | 60 |
| Ba-Zn Stabilisator, Typ Baerostab^{®} UBZ 760 XLP RF | 2 |

150 g PVC (Homopolymeres Suspensions-PVC, Markenname Inovyn^{®} 271 PC); 90 g Weichmacher-Zusammensetzung und 3 g Ba/Zn-Stabilisator, Markenname Baerostab^{®} UBZ 760 XLP RF wurden mit einem Handmixer bei Raumtemperatur vermischt. Die Mischung wurde anschließend auf einem ölbeheizten Labormischwalzwerk (Fa. Collin, Automatikwalzwerk Typ W250M, Durchmesser: 252 mm, Breite: 450 mm) plastifiziert und zu einem Walzfell verarbeitet. Die Temperatur der beiden Walzen betrug jeweils 180 °C; die Drehzahlen lagen bei 15 Umdrehungen/min. (vordere Walze) und 12 Umdrehungen/min. (hintere Walze); die Walzzeit betrug 5 Minuten. Der Walzenspalt wurde auf 0,5 mm eingestellt. Man erhielt so ein Walzfell mit einer Dicke von 0,53 mm. Das abgekühlte Walzfell wurde anschließend bei einer Temperatur von 190 °C und einem Druck von 150 bar innerhalb von 180 s auf einer Presse vom Typ "Laborplattenpresse 400 P" der Fa. Collin zu einer Weich-PVC-Folie mit einer Dicke von 0,50 mm verpresst. Unter Beibehaltung des Pressdrucks wurde die Pressfolie innerhalb von 10 Minuten auf ca. 40 °C abgekühlt.

### Il.c) Bestimmung der Shore A-Härte von Folien mit den offenbarungsgemäßen Weichmacher-Zusammensetzungen

Die Messung erfolgt in Anlehnung an die DIN EN ISO 868, Okt. 2003: Aus den wie unter Il.b) hergestellten Walzfellen werden insgesamt 22 Stück 49 x 49 mm große Folien ausgestanzt. Hierzu ist ein entsprechendes Stanzeisen zu verwenden um jeweils gleiche Foliengröße sicherzustellen. Diese werden in einen Pressrahmen (Maße 400 x 400 mm; Dicke 10 mm) luftblasenfrei eingelegt, in dem sich insgesamt 16 Kavitäten zur Herstellung von Shore-A Probekörpern befinden. Jede Kavität hat die Innenmaße von 50 x 50 mm. Nach dem Beschicken des Rahmens wird zwischen zwei hochglanzpolierten verchromten Messing-Pressblechen 400 x 400 x 2 mm gepresst auf einer Presse vom Typ "Laborplattenpresse 400 P" der Fa. Collin. Die Prüfkörper werden bei 185 °C und 200 bar für insgesamt 15 Minuten gepresst. Die abgekühlten Prüfkörper werden nun vor der Messung 7 Tage in einem Klimaraum bei 23 °C und ca. 50% Feuchte konditioniert. Für die Messung der Shore-A-Härte wird ein Durometer HDD-2 der Fa. Hildebrand verwendet. An einem Probekörper werden 10 Messwerte nach 15 s Eindringzeit gemessen.

### Il.d) Bestimmung der Folienflüchtigkeit von Folien mit den offenbarungsgemäßen Weichmacher-Zusammensetzungen

Für die Bestimmung der Folienflüchtigkeit wurden aus den unter Il.b) beschriebenen Pressfolien vier Einzelfolien (150 x 100 mm) ausgeschnitten, gelocht und gewogen. Die Folien wurden an einen rotierenden Stern in einen auf 130 °C eingestellten Heraeus-Trockenschrank Typ 5042 E gehängt. In dem Schrank wurde 18 mal pro Stunde die Luft gewechselt. Dies entspricht 800 L/h Frischluft. Nach 24 h in dem Schrank wurden die Folien entnommen und zurückgewogen. Der Gewichtsverlust in Prozent gibt die Folienflüchtigkeit der Weichmacher-Zusammensetzungen an.

### Il.e) Bestimmung der Verträglichkeit (Permanenz) von Folien mit den offenbarungsgemäßen Weichmacher-Zusammensetzungen

Für die Bestimmung der Verträglichkeit wurden aus den unter Il.b) beschriebenen Pressfolien 10 Prüfkörper (Folien) mit einer Größe von 75 x 110 x 0,5 mm ausgeschnitten. Die Folien wurden an der Breitseite gelocht, beschriftet und gewogen. Die so hergestellten Prüfkörper wurden anschließend auf ein Metallgestell aus nichtrostendem Material in ein Becken aus Glas gestellt. Um eine gegenseitige Beeinflussung zu vermeiden, dürfen nur Prüfkörper mit gleicher Zusammensetzung in einem Glasbecken gelagert werden. Die Glasbecken werden mit vollentsalztem Wasser bis zu einem Füllstand von etwa 3 cm befüllt. Dabei ist darauf zu achten, dass die Prüfkörper weitere 2 cm über der Wasseroberfläche sich befinden und das Wasser nicht berühren. Die anschließend luftdicht verschlossenen Glasgefäße werden dann in einen Wärmeschrank mit Innenraumtemperaturkontrolle gegeben. Die Prüfung erfolgt bei 70 °C und 100% rel. Luftfeuchtigkeit für eine Dauer von insgesamt 28 Tagen. In den Intervallen 1, 3, 7, 14 und 28 Tagen werden jeweils zwei Proben entnommen, und für 1 Std. frei hängend an der Luft klimatisiert. Danach wurden die Folien im Abzug mit Methanol gereinigt. Anschließend wurden die Folien für 16 h bei 80 °C in einem Trockenschrank (natürliche Konvektion) frei hängend getrocknet. Nach Entnahme aus dem Trockenschrank wurden die Folien für 1 Std. im Labor frei hängend konditioniert und anschließend gewogen. Als Prüfergebnis angegeben wurde jeweils der arithmetische Mittelwert der Gewichtsänderungen zu den Proben vor dem Einbringen in den Wärmeschrank.

Zusätzlich zur gravimetrischen Auswertung erfolgt eine visuelle Beurteilung der Folien. Dabei geht man nach der nachfolgenden Beurteilungstabelle vor:
0 = trockener Griff, die Folie ist glatt und trocken (beste Verträglichkeit)
1 = stumpfer Griff, die Folie ist noch trocken, eine geringe Weichmachermenge befindet sich auf der Oberfläche und ergibt einen stumpfen Griff. Fingerspuren sind sichtbar
2 = klebriger Griff, es ist schon merklich Weichmacher an der Oberfläche ausgetreten, Fingerspuren sind leicht und deutlich zu erkennen
3 = schwacher, trockener Belag; mit bloßem Auge erkennbar
4 = schwacher, flüssiger oder schmieriger Belag
5 = starker trockener Belag
6 = starker schmieriger Belag

### II.f) Bestimmung der HCl-Reststabilität von Folien mit den offenbarungsgemäßen Weichmacher-Zusammensetzungen

Die Bestimmung der HCl-Reststabilität erfolgt nach DIN EN 60811-405 (VDE 0473-811-405): Als Prüfgerät wird ein Metallblock-Thermostat von Liebisch Labortechnik bei einer Prüftemperatur von 200 °C verwendet. Es wird immer eine 3-fach Bestimmung vorgenommen. Ca. 50 mg der Walzfolien werden abgewogen, auf 3 cm Länge zugeschnitten und im unteren Teil des Glasröhrchens positioniert. Am oberen Ende des Glasröhrchens wird ein ca. 10 mm langer Indikatorpapierstreifen (Lackmusspapier) so positioniert, dass ca. 2 mm herausstehen. Die so präparierten Glasröhrchen werden in den Metallblock positioniert und die Zeit notiert, bis ein Farbumschlag in Richtung Rot auftritt. Aus den drei Messwerten der drei Proben wird der arithmetische Mittelwert gebildet.

### Il.g) Bestimmung der Kältebruchtemperatur von Folien mit den offenbarungsgemäßen Weichmacher-Zusammensetzungen

Die Prüfung der Kältebruchtemperatur erfolgt an Prüfkörper, die aus den unter II.b) hergestellten Pressfolien erhalten wurden. Die Prüfung erfolgt in Anlehnung an den Entwurf der DIN 53372 von 1981. Maße und Anzahl der Prüfkörper sind entsprechend den Vorgaben der DIN-Vorschrift (Länge von 60 mm, Breite von 15 mm, Dicke exakt 0,50 mm). Die Prüfkörper müssen vor der Prüfung min. 4 Tage bei Raumtemperatur gelagert werden. Entscheidender Unterschied dieser Prüfkonstruktion im Unterschied zum Entwurf der DIN 53372 besteht darin, dass die Hämmer nicht in freiem senkrechtem Fall auf die Prüfkörperschlaufen auftreffen. Stattdessen sind die Hämmer auf einer Welle befestigt und fallen nach dem Auslösen der Schlaggewichte im Kreisbogen aus gleicher Höhe (= Abstand zum Prüfkörper) auf die Prüfschlaufen herab. Hierbei werden jeweils sechs identische Prüfkörper in einer Reihe gleichzeitig geprüft. Die Kältetruhe wird auf eine zu erwartende Starttemperatur eingestellt und der Probenträger (Bombe) mit den Prüfkörpern eingesetzt. Zur Konditionierung der Prüfkörper werden diese pro Prüftemperatur 1 h temperiert. Zur Auswertung werden nur diejenigen Probeschlaufen als defekt gewertet die ganz in zwei oder mehr Teile zerbrochen sind. Um die Kältebruchtemperatur zu ermitteln, müssen mindestens eine 6er-Reihe Prüflinge als komplett zerbrochen, sowie eine 6er-Reihe als komplett erhalten bewertet werden. Das Temperaturintervall der durchzuführenden Prüfungen beträgt jeweils 5 °C. Die Berechnung der Kältebruchtemperatur erfolgt gemäß der Formel im Entwurf der DIN-Norm 53372 (1981).

### II.h) Bestimmung der Zugprüfeigenschaften von Folien mit den offenbarungsgemäßen Weichmacher-Zusammensetzungen

Diese Prüfung dient der Ermittlung der Parameter Bruchdehnung, Bruchspannung und 100% Modul. Dazu werden auf der Zugprüfmaschine Zwick / Z 2.5 Probekörper Typ 2 nach DIN EN ISO 527-3 gemessen. Die Probekörper sind 150 mm lang, 15 mm breit und ca. 0,50 mm dick. Die Probekörper werden mit Hilfe eines Stanzeisens aus den unter II.b) beschriebenen Pressfolien ausgestanzt. Vor der Prüfung werden die Prüfkörper 7 Tage im Klimaraum unter Normalklima konditioniert. Dabei ist zu beachten, dass zwischen Herstellung der Pressfolien und Durchführung der Zugprüfung genau 7 Tage vergehen. Die Konditionierung erfolgen bei 23 °C +/- 1,0 °C und 50% +/- 5 R.H. gemäß DIN EN ISO 291. Die Zugprüfungen erfolgen gemäß DIN EN ISO 527, Teil 1-3. Jede Messung besteht aus der Prüfung von 10 einzelnen Probekörpern. Die Messlänge von 100 mm ergibt sich aus der freien Einspannlänge des Probekörpers zwischen den Klemmbacken. Die Prüfgeschwindigkeit beträgt 100 mm/min. Innerhalb der Messlänge wird die Durchschnittsdicke aus 5 Einzelwerten ermittelt. Die Messung der Dehnung und des 100% Moduls erfolgt über die Änderung des Traversenweges.

Die Ergebnisse der anwendungstechnischen Prüfungen sind in Tabellen 6 und 7 gezeigt.

**Tabelle 6.**

| | | Verbindung I.1 | Verbindung I.2 | Verbindung I.6 | |
|---|---|---|---|---|---|
| Produkteigenschaft | Methode | (4-Oxoheptandisäure-di-(2-propylheptylester)) | (4-Oxoheptandisäure-di-(3,7-dimethyloctylester)) | (4-Oxoheptandisäure-di-isotridecylester) | 4-Oxoheptandisäure-di-(2-ethylhexylester)* |
| Reißfestigkeit [MPa] | DIN EN ISO 527, Teil 1+3 | 17,3 | 17,1 | 17,1 | 16,6 |
| Reißdehnung [%] | DIN EN ISO 527, Teil 1+3 | 355 | 353 | 311 | 377 |
| Spannungswert bei 100% Dehnung [MPa] | DIN EN ISO 527, Teil 1+3 | 7,6 | 7,8 | 9,3 | 6 |
| Shore A, 15 Sek | DIN EN ISO 868 | 78 | 79 | 89 | 71 |
| Kältebruchtemperatur [°C] | BASF-Methode | -43 | -43 | -48 | -45 |
| Folienflüchtigkeit [%] | BASF-Methode | 0,77 | 0,93 | 0,67 | 2,4 |
| HCl-Reststabilität [min] | **DIN** EN 60811-3-2 | 15 | 13,4 | 12 | 11,1 |

| | | | | | |
|---|---|---|---|---|---|
| * Vergleichsbeispiel | | | | | |

**Tabelle 7.**

| | | Verbindung I.1 | Verbindung I.2 | Verbindung I.6 | |
|---|---|---|---|---|---|
| Verträglichkeitsprüfung, 70 °C, 100% rel. Feuchtigkeit | Dauer | (4-Oxoheptandisäure-di-(2-propylheptylester)) | (4-Oxoheptandisäure-di-(3,7-dimethyloctylester)) | (4-Oxoheptandisäure-di-isotridecylester) | 4-Oxoheptandisäure-di-(2-ethylhexylester)* |
| Trockenwert | Nach 1 Tag | -0,42 | -0,58 | -1,7 | -0,47 |
| | Nach 3 Tagen | -0,54 | -0,62 | -2,3 | -0,64 |
| | Nach 7 Tagen | -0,54 | -0,88 | -3 | -0,7 |
| | Nach 14 Tagen | -0,62 | -0,86 | -3,5 | -0,95 |
| | Nach 28 Tagen | -0,84 | -1,11 | -4,6 | -1,0 |
| Visuelle Beurteilung | Nach 1 Tag | 2 | 2 | 6 | 0-1 |
| | Nach 3 Tagen | 2 | 4 | 6 | 1 |
| | Nach 7 Tagen | 2 | 4 | 6 | 1-2 |
| | Nach 14 Tagen | 2 | 4 | 6 | 1-2 |
| | Nach 28 Tagen | 4 | 6 | 6 | 1-2 |

| | | | | | |
|---|---|---|---|---|---|
| * Vergleichsbeispiel | | | | | |

## Patentansprüche

1. Verbindung der allgemeinen Formel (I) oder Gemisch von Verbindungen der allgemeinen Formel (I) 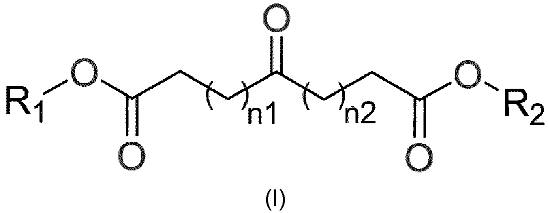 wobei
R₁ und R₂ unabhängig voneinander ausgewählt sind unter C₁₀-C₁₃-Alkyl, wobei zumindest ein Teil der Reste R₁ und/oder R₂ verzweigt ist, und
n1 und n2 unabhängig voneinander für 1, 2 oder 3 stehen.

2. Verbindung nach Anspruch 1, wobei n1 und n2 für 1 stehen.

3. Verbindung nach Anspruch 1 oder 2, wobei R₁ und R₂ unabhängig voneinander ausgewählt sind unter 2-Propylheptyl, 3,7-dimethyloctyl, iso-Decyl, iso-Undecyl, iso-Dodecyl, und iso-Tridecyl, bevorzugt 2-Propylheptyl, 3,7-dimethyloctyl, und iso-Tridecyl.

4. Verbindung nach einem der vorhergehenden Ansprüche, wobei R₁ und R₂ gleich oder konstitutionsisomer sind.

5. Weichmacher-Zusammensetzung, enthaltend mindestens eine Verbindung der allgemeinen Formel (I) nach einem der vorhergehenden Ansprüche, und mindestens einen weiteren Weichmacher, der von den Verbindungen der allgemeinen Formel (I) unterschiedlich ist.

6. Weichmacher-Zusammensetzung nach Anspruch 5, wobei der weitere Weichmacher ausgewählt ist unter
- Phthalsäuredialkylestern,
- Trimellitsäuretrialkylestern,
- Terephthalsäuredialkylestern
- Benzoesäurealkylestern,
- Dibenzoesäureestern,
- Hydroxybenzoesäureestern,
- Estern von gesättigten Monocarbonsäuren,
- Estern von ungesättigten Monocarbonsäuren,
- Estern von Hydroxymonocarbonsäuren,
- Estern von Dicarbonsäuren,
- Estern von gesättigten Hydroxydicarbonsäuren,
- Amiden und Estern von aromatischen Sulfonsäuren,
- Pentaerythritolestern,
- Alkylsulfonsäureestern,
- Glycerinestern,
- Isosorbidestern,
- Phosphorsäureestern,
- Citronensäurediestern und Citronensäuretriestern,
- Alkylpyrrolidonderivaten,
- 2,5-Furandicarbonsäureestern,
- 2,5-Tetrahydrofurandicarbonsäureestern,
- epoxidierten Pflanzenölen,
- epoxidierten Fettsäuremonoalkylestern,
- 1,2-Cyclohexandicarbonsäuredialkylestern,
- 1,3-Cyclohexandicarbonsäuredialkylestern,
- 1,4-Cyclohexandicarbonsäuredialkylestern,
- Polyestern aus aliphatischen und/oder aromatischen Polycarbonsäuren mit wenigstens zweiwertigen Alkoholen,
- weiteren Weichmachern, und
- Mischungen davon.

7. Weichmacher-Zusammensetzung nach Anspruch 5 oder 6, enthaltend die mindestens eine Verbindung der allgemeinen Formel (I) in einer Menge von wenigstens 10 Gew.-%, bevorzugt 30 bis 90 Gew.-%, stärker bevorzugt 50 bis 80 Gew.-%, und
den weiteren Weichmacher in einer Menge von 0 bis 90 Gew.-%, bevorzugt 10 bis 70 Gew.-%, stärker bevorzugt 20 bis 50 Gew.-%,
jeweils bezogen auf die Gesamtmasse der Weichmacher-Zusammensetzung.

8. Formmasse oder Plastisol, enthaltend mindestens eine Verbindung der allgemeinen Formel (I) nach einem der Ansprüche 1 bis 4, und mindestens ein Polymer.

9. Formmasse oder Plastisol nach Anspruch 8, wobei das Polymer ausgewählt ist unter einem Thermoplast, einem Elastomer, und Mischungen davon.

10. Formmasse oder Plastisol nach Anspruch 9, wobei
der Thermoplast ausgewählt ist unter
- Homo- oder Copolymeren, die mindestens ein Monomer in einpolymerisierter Form enthalten, ausgewählt unter C₂-C₁₀-Monoolefinen wie Ethylen oder Propylen, 1,3-Butadien, 2-Chlor-1,3-Butadien, Vinylalkoholen und deren C₂-C₁₀-Alkylestern, Vinylacetat, Vinylchlorid, Vinylidenchlorid, Vinylidenfluorid, Tetrafluorethylen, Glycidylacrylat, Glycidylmethacrylat, Acrylaten und Methacrylaten von C₁-C₁₀-Alkoholen, Vinylaromaten wie Styrol, Acrylnitril, Methacrylnitril, α,β-ethylenisch ungesättigte Mono- oder Dicarbonsäuren und Maleinsäureanhydrid,
- Homo- oder Copolymeren von Vinylacetalen, Polyvinylestern, Polycarbonaten, Polyestern, Polyethern, Polyetherketonen, thermoplastischen Polyurethanen, Polysulfiden, Polysulfonen, Polyethersulfonen, Polyacrylaten, Polymethylmethacrylaten, Polystyrolen, Polyvinylalkoholen, Polyvinylacetaten, Polyvinylbutyralen, Polyvinylchloriden, Polycaprolactonen, Cellulosealkylestern und Mischungen davon,
und
das Elastomer ausgewählt ist unter natürlichem Kautschuk, und synthetischem Kautschuk wie Polyisopren-Kautschuk, Styrol-Butadien-Kautschuk, Butadien-Kautschuk, Nitril-Butadien-Kautschuk, Chloropren-Kautschuk und Mischungen davon.

11. Formmasse oder Plastisol nach einem der Ansprüche 8 bis 10, zusätzlich enthaltend mindestens einen Zusatzstoff, ausgewählt unter Stabilisatoren, Gleitmitteln, Füllstoffen, Farbmitteln, Flamminhibitoren, Lichtstabilisatoren, Treibmitteln, polymere Verarbeitungsmitteln, Schlagzähverbesserern, optischen Aufhellern, Antistatika, Biostabilisatoren, Siliziumdioxid, Phenolharzen, Vulkanisiermitteln, Vernetzungsmitteln, Vulkanisierbeschleunigern, Vernetzungsbeschleunigern, Aktivatoren, Ölen, Alterungsschutzmitteln und Mischungen davon.

12. Verwendung der Formmasse oder des Plastisols nach einem der Ansprüche 8 bis 11 zur Herstellung von Formkörpern, Handschuhen, Folien, Tapeten, oder heterogenen Fußböden, oder zur Textilbeschichtung.

13. Verwendung der Verbindung der allgemeinen Formel (I) oder des Gemischs von Verbindungen der allgemeinen Formel (I) nach einem der Ansprüche 1 bis 4 als Weichmacher.

14. Verwendung nach Anspruch 13 als Weichmacher in einer Formmasse oder einem Plastisol.

## Claims

1. Compound of the general formula (I) or mixture of compounds of the general formula (I) 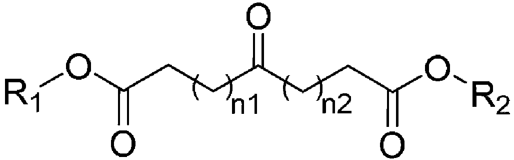 wherein
R₁ and R₂ are independently selected from C₁₀-C₁₃-alkyl,
wherein at least a portion of the radicals R₁ and/or R₂ is branched, and
n1 and n2 independently represent 1, 2 or 3.

2. Compound according to claim 1, wherein n1 and n2 represent 1.

3. Compound according to claim 1 or 2, wherein R₁ and R₂ are independently selected from 2-propylheptyl, 3,7-dimethyloctyl, iso-decyl, iso-undecyl, iso-dodecyl, and iso-tridecyl, preferably 2-propylheptyl, 3,7-dimethyloctyl, and iso-tridecyl.

4. Compound according to any of the preceding claims, wherein R₁ and R₂ are identical or are constitutional isomers.

5. Plasticizer composition containing at least one compound of the general formula (I) according to any of the preceding claims, and at least one further plasticizer which is different from the compounds of the general formula (I).

6. Plasticizer composition according to claim 5, wherein the further plasticizer is selected from
- phthalic acid dialkyl esters,
- trimellitic acid trialkyl esters,
- terephthalic acid dialkyl esters,
- benzoic acid alkyl esters,
- dibenzoic acid esters,
- hydroxybenzoic acid esters,
- esters of saturated monocarboxylic acids,
- esters of unsaturated monocarboxylic acids,
- esters of hydroxymonocarboxylic acids,
- esters of dicarboxylic acids,
- esters of saturated hydroxydicarboxylic acids,
- amides and esters of aromatic sulfonic acids,
- pentaerythritol esters,
- alkylsulfonic acid esters,
- glycerol esters,
- isosorbide esters,
- phosphoric acid esters,
- citric acid diesters and citric acid triesters,
- alkylpyrrolidone derivatives,
- 2,5-furandicarboxylic acid esters,
- 2,5-tetrahydrofurandicarboxylic acid esters,
- epoxidized vegetable oils,
- epoxidized fatty acid monoalkyl esters,
- 1,2-cyclohexanedicarboxylic acid dialkyl esters,
- 1,3-cyclohexanedicarboxylic acid dialkyl esters,
- 1,4-cyclohexanedicarboxylic acid dialkyl esters,
- polyesters of aliphatic and/or aromatic polycarboxylic acids with at least divalent alcohols,
- further plasticizers, and
- mixtures thereof.

7. Plasticizer composition according to claim 5 or 6, containing
the at least one compound of the general formula (I) in an amount of at least 10% by weight, preferably 30 to 90% by weight, more preferably 50 to 80% by weight, and
the further plasticizer in an amount of 0 to 90% by weight, preferably 10 to 70% by weight, more preferably 20 to 50% by weight,
in each case based on the total mass of the plasticizer composition.

8. Plastisol composition or plastisol containing at least one compound of the general formula (I) according to any of claims 1 to 4, and at least one polymer.

9. Plastisol composition or plastisol according to claim 8, wherein the polymer is selected from a thermoplastic polymer, an elastomeric polymer, and mixtures thereof.

10. Plastisol composition or plastisol according to claim 9, wherein
the thermoplastic polymer is selected from
homo- or copolymers containing at least one monomer in polymerized form, selected from C₂-C₁₀-monoolefins such as ethylene or propylene, 1,3-butadiene, 2-chloro-1,3-butadiene, vinyl alcohols and their C₂-C₁₀-alkyl esters, vinyl acetate, vinyl chloride, vinylidene chloride, vinylidene fluoride, tetrafluoroethylene, glycidyl acrylate, glycidyl methacrylate, acrylates and methacrylates of C₁-C₁₀-alcohols, vinyl aromatics such as styrene, acrylonitrile, methacrylonitrile, α,β-ethylenically unsaturated mono- or dicarboxylic acids and maleic anhydride,
- homo- or copolymers of vinyl acetals, polyvinyl esters, polycarbonates, polyesters, polyethers, polyether ketones, thermoplastic polyurethanes, polysulfides, polysulfones, polyether sulfones, polyacrylates, polymethyl methacrylates, polystyrenes, polyvinyl alcohols, polyvinyl acetates, polyvinyl butyrals, polyvinyl chlorides, polycaprolactones, cellulose alkyl esters and mixtures thereof,
and
the elastomeric polymer is selected from natural rubber, and synthetic rubber such as polyisoprene rubber, styrene-butadiene rubber, butadiene rubber, nitrile-butadiene rubber, chloroprene rubber and mixtures thereof.

11. Plastisol composition or plastisol according to any of claims 8 to 10, additionally containing at least one additive selected from stabilizers, lubricants, fillers, colorants, flame inhibitors, light stabilizers, blowing agents, polymeric processing aids, impact improvers, optical brighteners, antistatic agents, biostabilizers, silicon dioxide, phenolic resins, vulcanizing agents, crosslinking agents, vulcanization accelerators, crosslinking accelerators, activators, oils, anti-aging agents and mixtures thereof.

12. Use of the plastisol composition or the plastisol according to any of claims 8 to 11 for the production of molded articles, gloves, films, wallpapers, or heterogeneous floor coverings, or for textile coating.

13. Use of the compound of the general formula (I) or of the mixture of compounds of the general formula (I) according to any of claims 1 to 4 as a plasticizer.

14. Use according to claim 13 as a plasticizer in a plastisol composition or a plastisol.

## Revendications

1. Composé de formule générale (I) ou mélange de composés de formule générale (I) 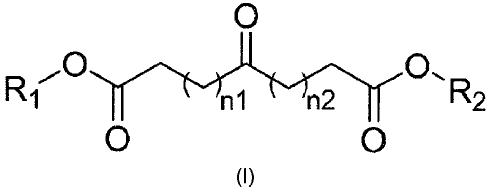 dans lequel
R₁ et R₂ sont choisis indépendamment l'un de l'autre parmi les alkyles en C₁₀-C₁₃, au moins une partie des radicaux R₁ et/ou R₂ étant ramifiée, et
n1 et n2 représentent indépendamment l'un de l'autre 1, 2 ou 3.

2. Composé selon la revendication 1, dans lequel n1 et n2 représentent 1.

3. Composé selon la revendication 1 ou 2, dans lequel R₁ et R₂ sont choisis indépendamment l'un de l'autre parmi le 2-propylheptyle, le 3,7-diméthyloctyle, l'iso-décyle, l'iso-undécyle, l'iso-dodécyle et l'iso-tridécyle, de préférence le 2-propylheptyle, le 3,7-diméthyloctyle et l'iso-tridécyle.

4. Composé selon l'une des revendications précédentes, dans lequel R₁ et R₂ sont identiques ou sont des isomères de constitution.

5. Composition plastifiante contenant au moins un composé de la formule générale (I) selon l'une des revendications précédentes, et au moins un autre plastifiant différent des composés de formule générale (I).

6. Composition plastifiante selon la revendication 5, dans laquelle l'autre plastifiant est choisi parmi
- les diesters dialkyliques de l'acide phtalique,
- les triesters trialkyliques de l'acide trimellitique,
- les diesters dialkyliques de l'acide téréphtalique,
- les esters alkyliques de l'acide benzoïque,
- les esters de l'acide dibenzoïque,
- les esters de l'acide hydroxybenzoïque,
- les esters d'acides monocarboxyliques saturés,
- les esters d'acides monocarboxyliques insaturés,
- les esters d'acides monocarboxyliques hydroxylés,
- les esters d'acides dicarboxyliques,
- les esters d'acides dicarboxyliques hydroxylés saturés,
- les amides et esters d'acides sulfoniques aromatiques,
- les esters de pentaérythritol,
- les esters d'acides alkylsulfoniques,
- les esters de glycérol,
- les esters d'isosorbide,
- les esters de l'acide phosphorique,
- les diesters et triesters de l'acide citrique,
- les dérivés d'alkylpyrrolidone,
- les esters de l'acide 2,5-furandicarboxylique,
- les esters de l'acide 2,5-tétrahydrofurandicarboxylique,
- les huiles végétales époxydées,
- les esters monoalkyliques d'acides gras époxydés,
- les diesters dialkyliques de l'acide 1,2-cyclohexanedicarboxylique,
- les diesters dialkyliques de l'acide 1,3-cyclohexanedicarboxylique,
- les diesters dialkyliques de l'acide 1,4-cyclohexanedicarboxylique,
- les polyesters d'acides polycarboxyliques aliphatiques et/ou aromatiques avec des alcools au moins difonctionnels,
- d'autres plastifiants, et
- leurs mélanges.

7. Composition plastifiante selon la revendication 5 ou 6, contenant ledit au moins un composé de formule générale (I) en une quantité d'au moins 10 % en poids, de préférence de 30 à 90 % en poids, plus préférablement de 50 à 80 % en poids, et
l'autre plastifiant en une quantité de 0 à 90 % en poids, de préférence de 10 à 70 % en poids, plus préférablement de 20 à 50 % en poids,
chacun rapporté à la masse totale de la composition plastifiante.

8. Masse de moulage ou plastisol contenant au moins un composé de formule générale (I) selon l'une des revendications 1 à 4, et au moins un polymère.

9. Masse de moulage ou plastisol selon la revendication 8, dans lequel le polymère est choisi parmi un thermoplastique, un élastomère et leurs mélanges.

10. Masse de moulage ou plastisol selon la revendication 9, dans lequel le thermoplastique est choisi parmi
- les homo- ou copolymères contenant au moins un monomère sous forme polymérisée, choisi parmi les monooléfines en C₂-C₁₀ telles que l'éthylène ou le propylène, le 1,3-butadiène, le 2-chloro-1,3-butadiène, les alcools vinyliques et leurs esters alkyliques en C₂-C₁₀, l'acétate de vinyle, le chlorure de vinyle, le chlorure de vinylidène, le fluorure de vinylidène,
le tétrafluoroéthylène, l'acrylate de glycidyle, le méthacrylate de glycidyle, les acrylates et méthacrylates d'alcools en C₁-C₁₀, les vinylaromatiques tels que le styrène, l'acrylonitrile, le méthacrylonitrile, les acides mono- ou dicarboxyliques à insaturation éthylénique en α,β et l'anhydride maléique,
- les homo- ou copolymères d'acétals vinyliques, les polyesters vinyliques, les polycarbonates,
les polyesters, les polyéthers, les polyéther-cétones, les polyuréthanes thermoplastiques, les polysulfures, les polysulfones, les polyéther-sulfones, les polyacrylates, les polyméthacrylates de méthyle, les polystyrènes, les alcools polyvinyliques, les acétates de polyvinyle, les polyvinylbutyrals, les polychlorures de vinyle, les polycaprolactones, les esters alkyliques de cellulose et leurs mélanges, et
l'élastomère est choisi parmi le caoutchouc naturel et les caoutchoucs synthétiques tels que le caoutchouc polyisoprène, le caoutchouc styrène-butadiène, le caoutchouc butadiène, le caoutchouc nitrile-butadiène, le caoutchouc chloroprène et leurs mélanges.

11. Masse de moulage ou plastisol selon l'une des revendications 8 à 10, contenant en outre au moins un additif choisi parmi les stabilisants, les lubrifiants, les charges, les colorants, les inhibiteurs de flamme, les stabilisants à la lumière, les agents gonflants, les auxiliaires de traitement polymères, les améliorants de la résistance aux chocs, les
agents d'azurage optique, les antistatiques, les biostabilisants, le dioxyde de silicium, les résines phénoliques, les agents de vulcanisation, les agents de réticulation, les accélérateurs de vulcanisation, les accélérateurs de réticulation, les activateurs, les huiles, les antioxydants et leurs mélanges.

12. Utilisation de la masse de moulage ou du plastisol selon l'une des revendications 8 à 11 pour la fabrication de corps moulés, de gants, de films, de papiers peints ou de revêtements de sol hétérogènes, ou pour le revêtement de textiles.

13. Utilisation du composé de formule générale (I) ou du mélange de composés de formule générale (I) selon l'une des revendications 1 à 4 en tant que plastifiant.

14. Utilisation selon la revendication 13 en tant que plastifiant dans une masse de moulage ou un plastisol.
